# EUROPEAN PATENT SPECIFICATION

(11) **EP 3 265 177 B1**
(45) Date of publication and mention of the grant of the patent: **14.06.2023**
(21) Application number: 16740867.3
(22) Date of filing: 22.01.2016
(51) Int. Cl.: A61P 17/02, A61P 17/18, A61K 31/353, A61K 36/82, A61K 47/14, A61K 47/26, A61K 47/22, A61K 9/06, A61K 9/10, A61K 9/00, A61K 47/06, A61K 47/10

(54) **FORMULATIONS OF HYDROPHILIC COMPOUNDS**
FORMULIERUNGEN VON HYDROPHILEN VERBINDUNGEN
PRÉPARATIONS DE COMPOSÉS HYDROPHILES

(30) Priority: 22.01.2015 US 201562106612 P; 22.01.2015 US 201562106609 P
(43) Date of publication of application: 10.01.2018
(73) Proprietor: Grey Pacific Labs, LLC, Rancho Dominguez, CA 90221 (US)
(72) Inventor: BURNISON, Chantal, Los Angeles, CA 90024 (US); METSELAAR, Josbert Maarten, 1411CT Naarden (NL); STAHL, Christopher, Los Angeles, CA 90024 (US)
(74) Representative: Zacco Denmark A/S
(86) International application number: PCT/US2016/014600
(87) International publication number: WO 2016/118907

(56) References cited:
- CN-A- 101 138 550
- US-A- 4 855 322
- US-A1- 2005 208 138
- US-A1- 2007 160 550
- US-A1- 2008 274 176
- US-A1- 2008 274 176
- US-A1- 2009 297 665
- US-A1- 2012 141 446
- US-A1- 2012 251 596
- US-A1- 2013 045 196
- US-A1- 2014 301 958
- STEPHEN HONG-WEI WU ET AL: "CHARACTERISTICS OF D-ALPHA-TOCOPHERYL PEG 1000 SUCCINATE FOR APPLICATIONS AS AN ABSORPTION ENHANCER IN DRUG DELIVERY SYSTEMS", PHARMACEUTICAL TECHNO, ADVANSTAR COMMUNICATIONS, EUGENE, OR, US, vol. 23, no. 10, 1 October 1999 (1999-10-01), XP009014624, ISSN: 0147-8087

## Description

### TECHNICAL FIELD

This application relates to formulations generally (e.g., pharmaceuticals or cosmetics), more particularly to formulations suitable for topical application comprising, for example, a hydrophilic compound or compounds in a continuous hydrophobic phase or phases, and associated methods of making and using such formulations.

### BACKGROUND

Many biologically active compounds (e.g., pharmaceutically and/or cosmetically active compounds) are highly hydrophilic. Formulating the biologically active hydrophilic compounds into a stable formulation with a hydrophobic carrier presents considerable challenges. The formulation may be produced by dispersing the biologically active hydrophilic compound in a continuous hydrophobic phase. However, such formulations often have poor stability, toxicity, and tend to phase separate, resulting in a poor shelf stability and inconsistent performance.
US 2012/141446 A1 relates to a composition for increasing the bioavailability of nutrients in humans and animals comprising an oil base emulsion into which nutrients selected from the group comprising omega fatty acids, vitamin D, Co Q10, green tea extract, and policosanol are added.
CN 101 138 550 A relates to a mixed micelle medicine preparation and a preparation method, which is prepared by the combination of various kinds of surface acting agents. The mixed micelle consists of the polyethylene glycol-12-hydroxy stearate and the other surface acting agents of one kind or various kinds. The other surface acting agents comprise phospholipid, VE Macrogol succinate, Macrogol-VE-carbonate and Macrogol-VE-succinate. In addition, the mixed micelle also comprises drugs, solvent, a stabilizer with or without other components and a PH conditioner. The amount of the polyethylene glycol-12-hydroxy stearate in the prescription is 4 percentage to 40 percentage, W/V: the amount of the phospholipid is 0 percentage to 30 percentage, W/V: the amount of the activator is 0 percentage to 30 percentage, W/V: the amount of the drug is 0.001 percentage to 10 percentage, W/V: the amount of the solvent is 0 percentage to 90 percentage, W/V. The medicine comprises the hydrophobicity drug and the lip solubility drug, but the medicine is not restricted by the both kinds of drugs. The present invention has the following advantages. Firstly, the preparation has good dilution stability, which can improve the defect in the present preparation and can meet the demanding for clinical drug administration. Secondly, the toxicity is low and the chemical stability is excellent.
XP 009014624 is directed to a study on the characteristics of D-α-tocopherol PEG 1000 succinate for application as an absorption enhancer in drug delivery systems.
US 2008/274176 relates to a particulate composition containing; a) 5 to 90% of at least one phosphatidyl choline component b) 5 to 90% of at least one diacyl glycerol component, at least one tocopherol, or mixtures thereof, and c) 1 to 40% of at least one non-ionic stabilising amphiphile, where all parts are by weight relative to the sum of the weights of a+b+c and where the composition contains particles of at least one non-lamellar phase structure or forms particles of at least one non-lamellar phase structure when contacted with an aqueous fluid. The invention additionally relates to pharmaceutical formulations containing such compositions, methods for their formation and methods of treatment comprising their administration.

### SUMNIARY OF THE DISCLOSURE

The present invention is defined in the independent claims, preferred embodiments are defined in the dependent claims. In the following there are described various formulations and their components, wherein those fromulations and components not falling under the definition of the claims are technological background for understanding the invention.

Described are formulations that may be topically applied and comprise a continuous hydrophobic phase and a hydrophilic phase. In embodiments a hydrophilic phase may be dispersed throughout the continuous hydrophobic phase. The formulation i`uay compris at least one hydrophilic compound. The hydrophilic phase can comprise a stabilizing component and a hydrophilic component normally insoluble in the continuous hydrophobic phase at room temperature absent emulsifier. **A** weight ratio of the stabilizing component to the hydrophilic compound(s) can be at least about **10:1.** The stabilizing component may comprises from about 8% to about 30% by weight D-a-tocopheryl polyethylene glycol **1000** succinate ("TPGS") based upon total weight of the stabilizing component, and from about 70% to about 92% by weight at least one emulsifier having a hydrophilic-lipophilic balance **(HLB)** value of less than 10. The stabilizing component may also comprise from 8 % to 30% by weight of a first, polysorbate, emulsifier having a hydrophilic-lipophilic balance **("HLB")** value of at least 10, and from 70% to 92% by weight of a second emulsifier having an HLB value of less than 10. The hydrophilic compound comprises a compound that is normally insoluble in the continuous hydrophobic phase at room temperature absent the stabilizing component. The hydrophilic compound is preferably biologically active or otherwise desireable. The hydrophilic component is substantially dissolved in the hydrophilic phase utilizing reverse micelles comprising TPGS and/or the one or more emulsifiers. If the first, polysorbate emulsifier(s) is used, the formulation comprises no more than about 4% by weight of the polysorbate emulsifier(s) having an HLB value of at least **10.**

The formulation may be a reverse micelle of the hydrophilic compound(s) in the continuous hydrophobic phase. The hydrophilic compound(s) may be present in the formulations in the form of reverse micelles that are stabilized by the stabilizing component. When including a stabilizing component, the formulation is preferably stable at room temperature, without agitation, for one week or more.

**T**he formulation may comprise a continuous hydrophobic phase, a hydrophilic phase, at least one hydrophilic compound, e.g, green tea extract, epigallocatechin gallate (EGCG), in an amount of at least about **0.1%** (preferably at least about 1%) by weight based upon total weight of the topical formulation, and a stabilizing component. The stabilizing component comprises D-a-tocopheryl polyethylene glycol **1000** succinate ("TPGS") in an amount from about 8% to about 30% by weight based upon total weight of the stabilizing component and/or at least one polysorbate emulsifier having an HLB value of less than **10** (preferably about 3 to 9), and at least one emulsifier having an HLB value of less than **10** (preferably about 3 to 8, and more preferably about 3 to 6). A weight ratio of the stabilizing component to the hydrophilic compound(s) in a TPGS-containing formulation is at least about **10:1.**

Topical formulations containing polysorbate comprise no more than about 4% by weight of the polysorbate emulsifier(s) having an HLB value of at least **10.**

The topical formulation comprises a continuous hydrophobic phase, at least one hydrophilic compound in an amount of at least **0.1%** (preferably greater than 1%) by weight based on the total weight of the topical formulation, and a stabilizing component. The stabilizing component may comprise D-a-tocopheryl polyethylene glycol **1000** succinate (TPGS) in an amount from about 8 % to about 3 0 % by weight based upon total weight of the stabilizing component, and at least one fatty acid monoester of sorbitan. The stabilizing component may also comprise or may alternatively comprise at least one polysorbate emulsifier(s) having an HLB value of at least 10, and at least one fatty acid monoester of sorbitan, wherein from about 8 % to about 30% by weight of the stabilizing component is polysorbate emulsifier(s) having an HLB value of at least **10.** The topical formulation comprises no more than about 4% by weight of the polysorbate emulsifier(s) having an HLB value of at least **10.**

The topical formulation comprises a continuous hydrophobic phase comprising mineral oil, squalane, or a mixture thereof, a hydrophilic phase comprising propylene glycol; at least about 0.1% by weight of green tea extract, e.g., epigallocatechin gallate (EGCG) compound based upon total weight of the formulation; and a stabilizing component. A weight ratio of the stabilizing component to the green tea extract compound(s) is at least about 10:1. The stabilizing component comprises D-a-tocopheryl polyethylene glycol 1000 succinate (TPGS) in an amount from about 8% to about 30% by weight based upon total weight of the stabilizing component, and one emulsifier having an HLB value of less than 6.

The topical formulation comprises a continuous hydrophobic phase comprising mineral oil, squalene, or a mixture thereof, a hydrophilic phase in an amount of at least about 1 % wt/wt the hydrophilic phase per the continuous hydrophobic phase, at least about 0.1% by weight of the epigallocatechin gallate (EGCG) compound based on total weight of the formulation, and a stabilizing component. The stabilizing component comprises at least one polysorbate emulsifier having an HLB value of at least 10, and at least one emulsifier having an HLB value of less than 10, wherein from about 8 % to about 30% by weight of the stabilizing componenet is polysorbate emulsifier(s) having an HLB value of at least 10. The topical formulation comprises no more than about 4% weight of the polysorbate emulsifier(s) having an HLB value of at least 10.

The topical formulation compnses a continuous hydrophobic phase comprising mineral oil, squalane, or a mixture thereof; a propylene glycol hydrophilic phase dispersed throughout said continuous hydrophobic phase , an epigallocatechingallate (EGCG) compound in an amount from 1.0% to 1.7% by weight based upon total weight of the topical formulation; and a stabilizing component in an amount of at least 15 % by weight based upon total weight of the topical formulation. The stabilizing component comprises TPGS in an amount of from 8 % to 30% by weight based upon total weight of the stabilizing component and at least one fatty acid monoester of sorbitan having an HLB value of less than 6. A weight ratio of the continuous hydrophobic phase to the stabilizing component is no more than 5:1, and the weight ratio of the stabilizing component to the EGCG compound is at least 10:1

Further particularly described is a topical formulation comprising a continuous squalane hydrophobic phase, a propylene glycol hydrophilic phase, an epigallocatechin gallate (EGCG) compound in an amount from about 1.0% to about 1.7% by weight based upon total weight of the topical formulation, and a stabilizing component in an amount of at least about 15 % weight based upon total weight of the topical formulation. A weight ratio of the continuous squalane hydrophobic phase to the propylene glycol hydrophilic phase is about 10:1. A weight ratio of the continuous squalane hydrophobic phase to the stabilizing component is no more than about 5:1. A weight ratio of the stabilizing component to the EGCG compound is at least about 10:1. The stabilizing component comprises D-a-tocopheryl polyethylene glycol 1000 succinate (TPGS) in an amount from about 8 % to about 30% by weight based upon total weight of the stabilizing component, and at least one fatty acid monoester of sorbitan having an HLB value of less than 6.

Further particularly described is a formulation comprising a continuous squalane hydrophobic phase, a propylene glycol hydrophilic phase, an epigallocatechin gallate (EGCG) compound in an amount from about **1.0%** to about **1.7%** by weight based on total weight of the formulation, and a stabilizing component. The weight ratio of the continuous squalane hydrophobic phase to the propylene glycol hydrophilic phase is at least about **10:1.** The stabilizing component comprises at least one polysorbate emulsifier having an HLB value of at least 10 and at least one fatty acid monoester of sorbitan emulsifier having an HLB value of less than 10. The polysorbate surfactant comprises from about 8 % to about 30% by weight based on total weight of the stabilizing component, and no more than about 3.5%by weight based on total weight of the formulation.

Further described is a topical formulation comprising a continuous hydrophobic phase, a hydrophilic phase, at least one hydrophilic compound substantially dissolved in the hydrophilic phase utilizing a stabilizing component comprising TPGS. The continuous hydrophobic phase comprises cyclopentasiloxane, squalane, dimethiconol , ethoxyheptyl bicyclooctanone, ubiquinone , various extracts, tetrahexyldecyl ascorbate, safflower seed oil, Oenorhem Biennis (evening primrose) oil, tocopherol linoleate/oleate , octyldodecyl citrate crosspolymer, tocopherol acetate, polydecene, caprylic/capric triglyceride, dimethicone , and corn oil. The topical formulation comprises at least about 0.8 mg of the hyd rophilic compound(s) per I g of the continuous hydrophobic phase.

Further described is a topical formulation that comprises a continuous hydrophobic phase, a hydrophilic phase comprising propylene glycol, at least one hydrophilic compound substantially dissolved in the hydrophilic phase, and a stabilizing component. The stabilizing component comprises at least one polysorbate emulsifier having an HLB value of at least 10 and, optionally, at least one emulsifier having an HLB value of less than 10. The continuous hydrophobic phase is a hydrophobic serum composed of cyclopentasiloxane, squalane, dimethiconol, ethoxyheptyl bicyclooctanone, ubiquinone, various extracts, tetrahexyldecyl ascorbate, safflower seed oil, Oenoz`hem Biennis (evening primrose) oil, tocopherol linoleate/oleate, octyldodecyl citrate crosspolymer, tocopherol acetate, polydecene, caprylic/capric triglyceride, dimethicone, and corn oil. The topical formulation comprises at least about 0.8 mg of the hydrophilic compound(s) per 1 g of the continuous hydrophobic phase.

Also described are methods of preparing the formulations and methods of treating a condition in a subject (6.g, a mammal) in need thereof, wherein the methods comprise topically administering the formulation to the subject.

### BRIEF DESCRIPTION OF THE FIGURES

**FIG. 1** depicts a schematic of the spherical reverse micelles formed in the formulations
**FIG. 2** depicts a schematic of the non-spherical reverse micelles formed in the formulations ; and
**FIG.** 3 depicts a schematic of the cylindrical reverse micelles formed in the formulations.

### DETAILED DESCRIPTION

Described is a formulation for application to a surface (6.g, a biological surface such as skin, endothelial tissue, mucous membrane(s) of the eye, vulva, anus, and nose, the inside of the mouth or other orifice, etc.), wherein the formulation includes a continuous hydrophobic phase, a hydrophilic phase, at least one hydrophilic compound (preferably biologically active) substantially dissolved in the hydrophilic phase, and a stabilizing component composed of D-a-tocopheryl polyethylene glycol **1000** succinate **(TPGS)** and/or at least one polysorbate emulsifier having an HLB value of at least **10** and, optionally, at least one emulsifier having an HLB value of less than 10. Also described is a method of incorporating at least one hydrophilic compound into a relatively hydrophobic vehicle, wherein the method includes utilizing a stabilizing component composed of D-a- tocopheryl polyethylene glycol **1000** succinate **(TPGS)** and/or at least one polysorbate emulsifier having an HLB value of at least 10, and, optionally, at least one emulsifier having an HLB value of less than 10, to stabilize a desired amount of the hydrophilic compound(s) in the hydrophobic vehicle.

The singular forms "a," "an," and "the" as used herein include plural referents unless the context clearly dictates otherwise. Thus, for example, reference to "a hydrophilic compound" includes reference to one or more of such compounds.

The term "emulsion" refers to a mixture of two or more liquids wherein one liquid is present as dispersed droplets in another liquid in which it is not soluble or miscible. The emulsion may be a microemulsion or nanoemulsion. The dispersed droplets in emulsion may be in the form of reverse micelles.

As used herein, the term "microemulsion" refers to a type of emulsion that is thermodynamically stable and comprises dispersed droplets having globule size of less than 100 nanometers.

The term "reverse micelle" is used herein according to its art-recognized meaning and includes all forms of reverse micelles, including, e.g., spherical reverse micelles, non-spherical reverse micelles, cylindrical reverse micelles, etc.

The term "stable" as used herein in connection with the formulations refers to formulations which exhibit no phase separation when kept, without agitation, at room temperature for one week or more.

The term "HLB" as used herein refers to a hydrophilic-lipophilic balance of a surface-active material (e.g., surfactant, emulsifier). An HLB value of 0 corresponds to a completely hydrophobic (i.a, lipophilic) molecule, and a value of **20** corresponds to a completely hydrophilic (i.a, lipophobic) molecule. The surface-active material having an HLB value of less than **10** is insoluble in water; whereas, the surface-active material having an HLB value of more than **10** is soluble in water.

As used herein, the term "substantially," in reference to a given property or condition, refers to a degree that one of ordinary skill in the art would understand that the given property or condition is met with a small degree of variance, such as within acceptable measuring tolerances.

A formulation comprises a continuous hydrophobic phase, a hydrophilic phase, at least one hydrophilic compound substantially dissolved in the hydrophilic phase, and a stabilizing component. The stabilizing component comprises D-a-tocopheryl polyethylene glycol 1000 succinate (TPGS). The formulation is stable for at least one week at room temperature without agitation. The formulation is composed of stabilized reverse micelles of the hydrophilic compound(s) in the continuous hydrophobic phase.

A formulation comprises a continuous hydrophobic phase, a hydrophilic phase, at least one hydrophilic compound substantially dissolved in the hydrophilic phase, and a stabilizing component. The stabilizing component comprises at least one polysorbate emulsifier having an HLB value of at least **10** and, optionally, at least one emulsifier having an HLB value of less than 10. The formulation is stable for at least one week at room temperature without agitation. The formulation is composed of stabilized reverse micelles of at least one hydrophilic compound in the continuous hydrophobic phase.

D-a-tocopheryl polyethylene glycol **1000** succinate (TPGS or vitamin E TPGS) is a water-soluble derivative of natural vitamin E, which is formed by esterification of vitamin E succinate with polyethylene glycol (PEG) **1000.** TPGS is a nonionic surfactant having an HLB value of 13.2 and a relatively low critical micelle concentration (CMC) of **0.02%** w/w. TPGS has an average molecular weight of 1,513 and completely dissolves in water. TPGS is an FDA approved pharmaceutically safe adjuvant, and its oral **LD50** is reported to be more than 7 g/kg for young adult rats of both sexes. See, eg, Guo et al., The Applications of Vitamin E TPGS in Drug Delivery, Eur. J. Pharmc. Sci. 49 (2013), 175-186.

The formulation comprises a continuous hydrophobic phase, a hydrophilic phase, at least one hydrophilic compound substantially dissolved in the hydrophilic phase, and a stabilizing component. **A** weight ratio of the stabilizing component to the hydrophilic compound(s) is at least about 10:1. The stabilizing component comprises D-a-tocopheryl polyethylene glycol 1000 succinate (TPGS) in an amount from about 8% to about 30% by weight based upon total weight of the stabilizing component, and at least one emulsifier having an HLB value of less than 10 (preferably about 3 to 8, and more preferably about 3 to 6).

The formulation may comprise a continuous hydrophobic phase, a hydrophilic phase, at least one hydrophilic compound substantially dissolved in the hydrophilic phase, and a stabilizing component composed of at least one polysorbate emulsifier having an HLB value of at least 10 and at least one emulsifier having an HLB value of less than 10. About 8 % to about 30% by weight of the stabilizing component is polysorbate emulsifier(s) having an HLB value of at least 10. The formulation comprises no more than about 4% by weight of the polysorbate emulsifier(s) having an HLB value of at least 10. The formulation may be in the form of nanoemulsion of the hydrophilic compound(s) in the continuous hydrophobic phase.

The formulation may comprise a continuous hydrophobic phase, a hydrophilic phase, at least one hydrophilic compound substantially dissolved in the hydrophilic phase, and a stabilizing component composed of at least one polysorbate emulsifier having an HLB value of at least 10. The formulation may be in the form of microemulsion of the hydrophilic compound(s) in the continuous hydrophobic phase.

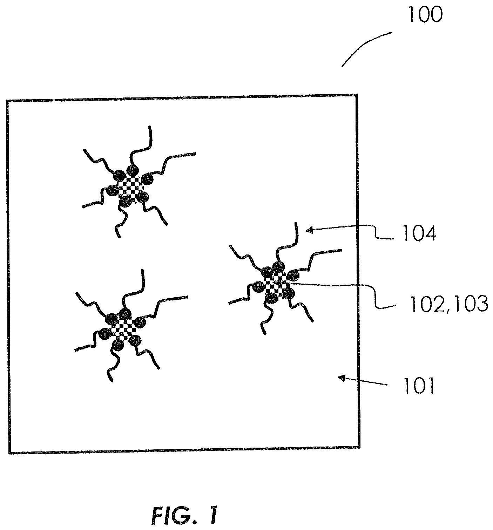

As depicted in FIG. 1, a formulation 100 that may be topically applied comprises a continuous hydrophobic phase 101, a hydrophilic phase 102, at least one hydrophilic compound 103 substantially dissolved in the hydrophilic phase 102, and a stabilizing component 104. In FIG. 1, due to their hydrophilicity, the hydrophilic phase 102 and the hydrophilic compound(s) 103 may form spherical reverse micelles dispersed in the continuous hydrophobic phase 101. The stabilizing component 104 is composed of surface-active compounds to stabilize the dispersed spherical reverse micelles in the continuous hydrophobic phase 101.

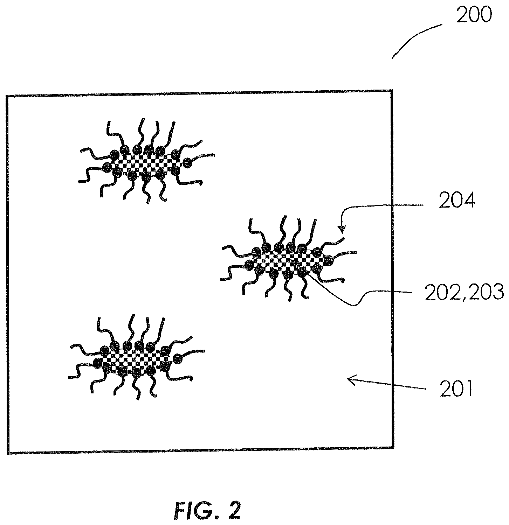

As depicted in FIG. 2, a formulation 200 that may come into contact with a subject comprises a continuous hydrophobic phase 201, a hydrophilic phase 202, at least one hydrophilic compound 203 substantially dissolved in the hydrophilic phase 202, and a stabilizing component 204. The hydrophilic phase 202 and the hydrophilic compound(s) 203 form dispersed non- spherical reverse micelles when they are mixed with the continuous hydrophobic phase 101. The stabilizing component 204 enhances the stability of the dispersed non-spherical reverse micelles in the continuous hydrophobic phase 201.

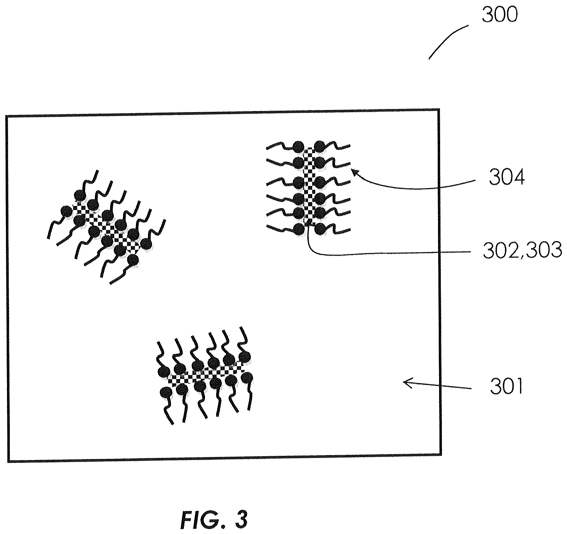

As depicted in FIG. 3, a formulation 3 00 that may come into contact with a subject comprises a continuous hydrophobic phase 301, a hydrophilic phase 302, at least one hydrophilic compound 303 substantially dissolved in the hydrophilic phase 302, and a stabilizing component 304. Upon mixing a solution of the hydrophilic compound(s) 303 in the hydrophilic phase 302 with the continuous hydrophobic phase 301, cylindrical reverse micelles are formed and dispersed in the continuous hydrophobic phase 301. The stabilizing component 304 facilitates the dispensability and enhances the stability of cylindrical reverse micelles in the continuous hydrophobic phase 301.

Although **FIGS.** 1-3 show three different structures of reverse micelles, it is understood that other structures of reverse micelles may be formed in the formulations. Such differently configured micelles may be present in the same formulation or at different times or temperatures of the formulation. A particular formulation hereof may contain all such depicted reverse micelles.

### Hydrophilic Compounds

Various biologically active (e.g., pharmaceutically active and/or cosmetically active) hydrophilic compounds may be used. Alternatively or in addition, hydrophilic compounds such as scents, or colorants, or insect repellants may be used, which are not biologically active in the subject. Examples of biologically active hydrophilic compounds may include, but not be limited to, green tea extract, grape seed extract, fruit extract, polyphenol compounds, or mixtures thereof.

The hydrophilic compound includes green tea extract. Green tea itself though is a complex composition of polyphenols. Green tea extract is derived from green tea leaves (Camellia sinensz's), and contains various antioxidant ingredientsmmainly green teacatechins (GTC). GTC itself comprises four major epicatechin derivatives; 1.€., epicatechin(EC), epigallocatechin (EGC), epicatechin gallate (ECG), and epigallocatechin gallate (EGCG). Other components of green tea include flavonoids, such as kaempferol, quercetin, and myricetin. Studies suggest that green tea polyphenols may help prevent skin cancer with direct (topical) application to the skin. See, e.g., Katiyar et al., "Green tea and skin," Arch Dermatol. 1362989-994 (2000). Evidence also exists that green tea constituents helps protect the skin from sun damage. See, cg,

Katiyar et a1 ., "Polyphenolic antioxidant (-)- epigallocatechin-3-ga11ate from green tea reduces UVB-induced inflammatory responses and infiltration of leukocytes in human skin," Photochem Photobiol. 69: "Double-blinded, placebo-controlled trial of green tea extracts in the clinical and histologic appearance of photoaging skin," Dermatol Surg. 3 1 :855m860 (2005). Furthermore, one percent (1% w/w) epigallocatechin gallate (EGCG-derived from green tea) topical formulations has been suggested as being useful for treating acne.

The hydrophilic compound includes at least one polyphenol compound.

The term "polyphenol" has been defined differently. For example, under one widely accepted definition, the White-Bate-Smith-Swain-Haslam (WBSSH) definition, polyphenols are generally moderately water-soluble compounds, typically with molecular weights of about 500 Da to about4000 Da, having, 6.g, more than 12 phenolic hydroxyl groups, and about 5 to 7 aromatic rings per **1000** Da. Under the proposed "Quideau definition" of polyphenols , "[t]he term 'polyphenol' should be used to define compounds exclusively derived from the shikimate/phenylpropanoid and/or the polyketide pathway, featuring more than one phenolic unit and deprived of nitrogen-based functions." As used herein, the term "polyphenol" is to be used inclusively.

Non-limiting examples of polyphenol compounds include epigallocatechin gallate (EGCG), epigallocatechin (EGC), epicatechin (EC), epicatechin gallate (ECG), and catechin.

The hydrophilic compound may include those compounds, vailable from, e.g, Silab S.A. of Saint Viance, France, selected from the group consisting of: hydrolyzed pepper fruit extract (Piper nigrum) (Retilactyl D^{®}); hydrolyzed soy protein (Ridulisse C^{®}, Ridulisse C^{®} Bio, Pro-Coll-One+C^{®}, or Raffermine^{®}); peppermint extract Menthol piperz'ra ) (Calmiskin'B) ; lotus (Neiumbo nucz'fera) (Pro-Sveltyl^{®}); coriander (Coriandrum sarivum) (Affiness) compound that may also contains orange extract); Verbena oficz'nab's extract (Vitalayer^{®}); and Heliam`hus anmms (sunflower) seed extract (Antiglyskin^{®} orHelioxineGE).

The hydrophilic compound may include, for example, (supplier in parenthesis): Alistin (DD Chemco), Chlorellagen DP (DD Chemco), Depollutine (Devereaux), Derm SRC (DD Chemco), Dragosine (Symrise), Ellagi-C (Devereaux), Hydrolite *S P* (Symrise), IBR Dormins (Israeli Biotechnology Research, Ltd) Kaden berry extracts (Symrise), Matrixyl synthe 6 (Croda), Matrixyl **3000** (Croda), Micromeral (BASF), Milk Peptide Complex (Devereaux), Mitostime L (DD Chemco), Minythis (DD Chemco) Muciliance fruit (Devereaux), Phytosan-K (Devereaux), Pholsine (DD Chemco), Pinoxide (DD Chemco), Quicklift (BASF), Scopariane BPC (DD Chemco), Seanergilium (BASF), Siiox Apple (BASF), Slimbuster L (DD Cbemco), SymMatrix (Tri-K), SymPeptide (Symrise), SymVital (Tri-K),Thiotaineaifi)L-ergothioneine (DD Chemco), or Ursolisome (BASF).

Further examples of the hydrophilic compounds may include, but are not limited to, those available from, 6.3., Silab SA. of Saint Viance, France, such as those selected from the group consisting of Saccharomy66s 66r6visia6 extract, Withania somnifera root extract, butylene glycol and hydrolyzed Coriandrum sativum fruit extract and Citrus aurantium dulcis (orange) fruit extract, Helianthus annuus (sunflower) seed extract, Taraxacum oflicinale (dandelion) extract, hydrolyzed Viola tricolor extract, Pyrus malus (apple) fruit extract (6.3., Bioprotectyl^{®}), hydrolyzed C6l0sia cristata extract and hydrolyzed Prunella vulgaris extract, hydrolyzed Citrus aurantium dulcis fruit extract, hydrolyzed Candida saitoana extract (6.3., Celldetox^{®}), Lindera stijzchnifolia root extract, Triticum vulgar6 (wheat germ) extract, Secal6 66r6al6 (rye) seed extract, hydrolyzed sesame extract, Tropaeolum majus flower extract, Spiraea ulmaria extract, hydrolyzed sweet almond protein, Salix Alba (willow) leaf extract (6.3., Astressyl^{®}), Mentha piperita (peppermint) extract, T*r*iticum vulgare (wheat) germ extract, butylene glycol and Butyrospermum parkii (shea butter) seedcake extract, hydrolyzed lupine protein octenylsuccinate, hydrolyzed Cucurbita pepo (pumpkin) seedcake, hydrolyzed Opuntia ficus indica flower extract, Medicago sativa (alfalfa) seed extract and hydrolyzed lupine protein, Lentinus 66loa'6s extract, hydrolyzed Ceratonia siliqua seed extract, Gossypium hirsutum (cotton) extract, H6lianthus anauus (sunflower) seed extract, Jasminum officinal6 (jasmine) flower extract (6.3., Helisun^{®}), hydrolyzed Manihot 6scu16nta tuber extract, butylene glycol, and Iris florentina root extract, hydrolyzed wheat protein, hydrolyzed Myrtus communis leaf extract, butylene glycol and Boerhavia dzflusa root extract, Cynara scolymus (artichoke) leaf extract, yeast extract, hydrolyzed rice protein, Nasturtium officinal6 extract, Avena sativa (oat) kernel extract, Tropaeolum majus flower/leaf/stem extract, Lens esculenta (lentil) seed extract (including oligosaccharides thereof), Cyperus 6scul6ntus tuber extract, Prunus amygdalus dulcis (sweet almond) seed extract, hydrolyzed soy fiber, Pichia anomala extract, butylene glycol, and N6lumb0 nucifera leaf extract, yeast extract, butylene glycol, and Artemisia abrotanum extract, hydrolyzed soy flour, Castanea sativa (chestnut) seed extract, propylene glycol, and Citrus aurantium amara (bitter orange) flower extract, hydrolyzed pepper fruit extract, hydrolyzed soy protein, butylene glycol, and Spiraea ulmaria extract, hydrolyzed linseed extract, butylene glycol, and Peumus boldus leaf extract, red algae (Kappaphycus alvareziz) galactans, Pichia anomala (including biopeptides thereof) extract, sulfated galactans of Hypa6a muscfiormis ( 6.3., Deglysome ^{®}), Glycine rota hydrolyzed soy protein (6.3., Ridulisse C. and/or Pro-Coll-One+CED), Oryza sativa extract (6.3., Nutripeptides ^{®}), Cyperus esculentus tuber extract (e.g., Papilactyl D^{®}), Piper nigrum hydrolyzed pepper fruit extract (e.g, Retilactyl DO), Verbena Officmalis extract ( e.g, VitalayerO), butylene glycol and Peumus boldus leaf extract, butylene glycol and Peumus boldus leaf extract, seed extract (e.g, SMS Anti-wrinkleO), hydrolyzed Cicer seed extract, Trz'z'icum Valgare (wheat) germ extract, hydrolyzed pea, butylene glycol and Silybum Marianam extract, hydrolyzed lupine protein, Kappaphycus alvareziz' (red algae) extract, Trz'z`icumvulgare (wheat) protein, yeast extract (e.g, Toniskin^{®} and/or Unflamagyl^{®}), Cz`chorz`ummlybus (chicory) root extract, Prunes persica (peach) leaf extract, Verbena os'cmalz's extract, Medicago sativa (alfalfa) extract, Paeom'a albiflora root extract, Pair/maria palmara extract, hydrolyzed millet, butylene glycol and triethanolamine and Punica granarum extract, glycerin and Prunes amygdalus dulcz's (sweet almond) extract, hydrolyzed sweet almond seedcake, butylene glycol and Famarz'a ofiicmab's extract, butylene glycol and Camellia sir/semis leaf extract, soytrimonium chloride, or combinations of any thereof.

Moreover, examples of the hydrophilic compounds may include, but not be limited to, proteins, peptides, nucleic acids, or hydrophilic drugs (characterized chemically by having a relatively low octanol/water partition coefficient) such as vancomycin, phenobarbital, antibiotics such as ampicillin, streptomycin, aminoglycosides, and the penicillins, theophylline, polysaccharides, non- ADEK vitamins, digoxiri, atenolol, paclitaxel , 5-fluorouracil, iriuliri, lithium, ara-C, gemcitabine, propranolol, tramadol, diltiazem, etc.

The amount of, e.g, hydrophilic compounds in the formulations depends on various factors, such as the desired dosage of the hydrophilic compounds, the types of hydrophilic solvents, the types of continuous hydrophobic phase, and the types of stabilizing components. It is also appreciated that the amount of hydrophilic compounds in the formulations may also depend on the nature of the conditions for which treatment is required.

The formulation may comprise at least about 0.1% by weight, preferably at least about 1% by weight, of the hydrophilic compound(s) based upon total weight of the formulations. The formulations may comprise at least about 0.1% gig of the hydrophilic compound(s) per the continuous hydrophobic phase. Thus, for example the amount of the hydrophilic compound can vary from about 0.01%, 0.05%, 0.1%, 0.2%, 0.3%, 0.4%, 0.5% or 1.0% to about 1.5%, 2.0%, 3.0%, 4.0%, 5.0%, 6.0%, 10% or even higher.

### Hydrophilic Phase

The hydrophilic phase may include one or more hydrophilic solvents. Various nontoxic pharmaceutically and/or cosmetically acceptable hydrophilic solvents may be used to substantially dissolve the hydrophilic compound(s). Examples of such hydrophilic solvents may include, but are not limited to, water, ethanol, dimethyl sulfoxide (DMSO), propylene glycol (PG), and polyethylene glycol (PEG) at different molecular weights (e.g., PEG 400 and PEG 800). These hydrophilic solvents can sometimes serve as humectants for the hydrophilic compounds, such as a pharmaceutically active ingredient, to be included in the formulation. Thus, other humectants known to those having ordinary skill in the art can also be incorporated into the hydrophilic phase.

The hydrophilic solvent may be present in the formulations in an amount sufficient to provide a stable dispersed phase of the hydrophilic compound in the continuous hydrophobic phase. Thus, for example, the amount of hydrophilic solvent can vary from about 0.5%, 1.0%, 2.0%, 3.0%, 4.0% or 5.0% to about 7.0%, 8.0%, 9.0%, 10%, 15%, 20%, 25%, 30%, 35%, 40%, 45%, 50%, 55%, 60% or65%.

### Stabilizing Component

The stabilizing component may comprise TPGS and/or at least one polysorbate emulsifier having an HLB value of at least 10 ("polysorbate emulsifier") and, optionally, at least one emulsifier having an HLB value of less than 10 (preferably from 3 to 8, and more preferably form 3 to 6).

A weight ratio of the stabilizing component to the hydrophilic compound(s) is at least about 10:1.

The stabilizing component compnses at least one polysorbate emulsifier having an HLB value of at least 10 (preferably from about 12 to about 20), and the emulsifier having an HLB value of less than 10. About 8% to about 30% by weight of the stabilizing component is polysorbate emulsifier(s) having an HLB value of at least 10. The formulation comprises no more than about 4% by weight of the polysorbate emulsifier(s) having an HLB value of at least 10.

The polysorbate emulsifier(s) having an HLB value of at least 10 accounts for about 8 % to about 30% by weight based on total weight the stabilizing component, and no more than about 4 % by weight based on total weight of the formulation. Examples of the polysorbte emulsifiers (i.e., PEG sorbitan fatty acid esters ay include, but not be limited to, PEG-2O orbitan monolaurate having an HLB value of 17 (e.g. polysorbate 20, TWEEN 20 from Croda, Inc.) PEG-20 sorbitan monopalmitate having an HLB value of 16 (e.g. TWEEN 40 from Croda, Inc.); PEG-20 sorbitan monostearate having an HLB value of 15 (e.g. TWEEN 60 from Croda, Inc.); and PEG-20 sorbitan monooleate having an HLB value of 15 (e.g. polysorbate 80, TWEEN 80 from Croda, Inc.)

The formulations comprise the stabilizing component in an amount of at least about 10% by weight based upon total weight of the formulations. The formulation includes PEG-20 sorbitan monooleate having an HLB value of 15 (e.g. polysorbate 80, TWEEN 80 from Croda, Inc.) as the polysorbate emulsifier having an HLB value of at least 10.

A weight ratio of the continuous hydrophobic phase to the stabilizing component is no more than about 25 :1. Thus, for example, the ratio can be from about 1:1, 2:1, 3:1, 4:1 or 5:1 to about 5:1, 6:1, 7:1, 8:1. 9:1. 10:1, 15:1 20:1 or 25:1.

The stabilizing component comprises TPGS in an amount from about 0.1%, 0.5%, 1.0%or2.0% to about 2.0%, 3.0%, 5.0%, 10%, 20% or 30%by weight based upon total weight of the stabilizing component, and at least one emulsifier having an HLB value of less than 10.

The emulsifier having an HLB value of less than 10 ("low HLB emulsifier") may include a cationic surfactant, an anionic surfactant, an amphoteric surfactant, a non-ionic surfactant, or a mixture thereof. The low HLB emulsifier may include a surfactant having an HLB value of about 3 to about 9.

The low HLB emulsifier may include a compound selected from the group consisting of fatty acid monoesters of glycerol, fatty acid diesters of glycerol, fatty acid monoesters of propylene glycol, fatty acid diesters of propylene glycol, fatty acid monoesters of sorbitan, fatty acid diesters of sorbitan, fatty acid triesters of sorbitan, and triglyceride.

The emulsifiers having an HLB value of less than 10 ("low HLB emulsifiers") may include a compound selected from the group consisting of fatty acid monoesters of glycerol, fatty acid diesters of glycerol, fatty acid monoesters of propylene glycol, fatty acid diesters of propylene glycol, fatty acid monoesters of sorbitan, and fatty acid diesters of sorbitan.

Examples of the fatty acid mono-and di-esters of glycerols may include, but not be limited to, IMWITOR^{®} 742 surfactant having an HLB value of less than 10, which is a mixture of mono- and di-glycerides of caprylic and capric acids from Sasol Olefins and Surfactants GmbH; IMWITOR^{®} 988 surfactant having an HLB value of about 5 to 6, which is a mixture of mono- and di-glycerides of caprylic acid from Sasol Olefins and Surfactants GmbH; IMWITOR^{®} 308 surfactant having an HLB value of about 5 to 6, which is a mixture of mono- and di-glycerides of caprylic acid with more than about 80% monoglycerides from Sasol Olefins and Surfactants GmbH, IMWITOR^{®} 191 surfactant having an HLB value of about 4.4, which is glyceryl monostearate from Huls AG/Huls America, CAPMUL^{®} MCM surfactant having an HLB value of about 5 to 6; which is monoglyceride of caprylic and capric acid from ABITEC Corporation; CAPMUL^{®} GMO-SO surfactant having an HLB value of less than 10; which is glyceryl monooleate from ABITEC Corporation; CAPMUL^{®} GDL surfactant having an HLB value of about 3 to 4; which is glyceryl dilaurate from ABITEC Corporation; MAISINE^{®} 35-1surfactant having an HLB value of about 3 to 4; which is glyceryl monolinoleate from Gattefosse Corporation; and PECEOLTM surfactant having an HLB value of about 3 to 4; which is glyceryl monooleate from Gattefosse Corporation.

Examples of the fatty acid mono- and di-esters of propylene glycol may include; but not be limited to; CAPTEX^{®} 200 having an HLB value of more than 6; which is propylene glycoldicaprylate/dicaprate from ABITEC Corporation; Miglyo^{®} 840 surfactant having an HLB value of about more than 6; which is propylene glycol dicaprylate/dicaprate from Sasol Olefins and Surfactants GmbH; Neobee^{®} **M-20** surfactant having an HLB value of about more than 6; which is propylene glycol dicaprylate/dicaprate from Stepan Company; LAUROGL YCOLTM surfactant having an HLB value of about less than 10; which is propylene glycol monolaurate from Gattefosse Corporation; MIRPYL^{®} surfactant having an HLB value of about less than 10; which is propylene glycol monolaurate; CAPMUL. P68 surfactant having an HLB value of about 4; which is propylene glycol monocaprylate with up to about 5 % propylene glycol dicaprylate from ABITEC Corporation.

Examples of the fatty acid mono-and di-esters of sorbitan may include; but not be limited to; sorbitan monooleate such as SPANTM 80 surfactant having an HLB value of about 4.3 from Croda Inc.; sorbitan monopalmitate such as SPANTM 4O surfactant having an HLB value of about 6.7 from Croda Inc.; sorbitan monolaurate such as SPANTM 20 surfactant having an HLB value of about 8.6 from Croda Inc; sorbitan monostearate such as SPAN]M 60 surfactant having an HLB value of about 4.7 from Croda Inc; sorbitan sesquioleate such as SPANTM 83 surfactant from Croda Inc.; sorbitan monoisostearate such as SPANTM 120 surfactant from Croda Inc.; and sorbitan sesquistearate such as NIKKOL "R3SS-15 surfactant having an HLB value of about 4.2 from Nikkol Chemicals Co.; Ltd.

The triglycerides may be medium chain (C6-C12) triglycerides; long chain (C14-C20) triglycerides; or a mixture thereof. Examples of the medium chain (C6-C12) triglycerides may include; but not be limited to; fractionated coconut oils; MIGLYOL^{®} 812 compound having an HLB value of about 1 from Sasol Olefins and Surfactants GmbH that contains 56% caprylic (C8) and 3 6% capric (C10) triglycerides; MIGLYOL^{®} 810 compound having an HLB value of about 1 from Sasol Olefins and Surfactants GmbH that contains 68% caprylic (C8) and 28% capric (C10) triglycerides; NEOBEE^{®} M5 compound having an HLB value of about 1 from Stepan Company; which is caprylic/capric triglyceride; CAPTEX^{®} 300 and 355 compounds having an HLB value of about 1 from ABITEC Corporation that is glyceryl tricaprylate/tricaprate; and CRODAMOLTM GTCC compound having an HLB value of about 1 from Croda Inc that is glyceryl tricaprylate/tricaprate. Examples of the long chain triglycerides (Cl4-C20) triglycerides may include, but not be limited to, vegetable oils such as safflower, corn, soybean, olive, cotton seed, sunflower seed, arachis, palm and rapeseed oils.

The formulation includes a fatty acid monoester sorbitan, particularly sorbitan monoleate (e.g, SPANTM80 surfactant having an HLB value of about 4.3), as at least one of the low HLB emulsifier.

**T**he stabilizing component in the formulation comprises **TPGS** emulsifier and at least one low HLB emulsifier including sorbitan monooleate.

The stabilizing component m the formulation may comprise at least one low HLB emulsifier including sorbitan monooleate, and at least one polysorbate emulsifier having an HLB value of at least 10 including **PEG-20** sorbitan monooleate.

### Continuous Hydrophobic Phase

The continuous hydrophobic phase may include one or more pharmaceutically and/or cosmetically acceptable hydrophobic solvents.

Non-limiting examples of hydrophobic solvents include, but not be limited to, mineral oil, vegetable oil, paraffin , polysiloxane , cyclopentasiloxane , dimethiconol, squalane, hemisqualane and mixtures thereof. The continuous hydrophobic phase may include at least a mixture of cyclopentasiloxane and dimethiconol, such as Cosmetic Fluid **1585-0H** from Chemsil Silicones, Inc.

The continuous hydrophobic phase of the formulations does not include any triglyceride.

The continuous hydrophobic phase may include oleaginous ointment bases, absorption ointment bases, water/oil emulsion ointment bases, oil/water emulsion ointment bases, and water-miscible ointment bases such as white petrolatum , white ointment, hydrophilic petrolatum , anhydrous lanolin, AquabaseTM, Aquaphor ^{®}, Polysorb^{®}, cold cream type, hydrous lanolin, rose water ointment, HydrocreamTM, Eucerin^{®}, Nivea^{®}, DermabaseTM, Velvachol^{®}, Unibase^{®}, PEG ointment, and PolybaseTM.

The continuous hydrophobic phase may be serum/gel that includes cyclopentasiloxane, squalane, dimethiconol, ethoxyheptyl bicyclooctanone (ETHOCYN ^{i532),} ubiquinone , various extracts, tetrahexyldecyl ascorbate, safflower seed oil, Oenoz`hera biennis (evening primrose) oil, tocopherol linoleate/oleate, octyldodecyl citrate crosspolymer, tocopherol acetate, polydecene, caprylic/capric triglyceride, dimethicone, and corn oil.

ETHOCYN^{®} is a bicyclooctane DHT competitive inhibitorIcollagen suppressing compound, which could be topically applied with the formulation . Other collagen suppressing compounds could also or alternatively be incorporated into the formulation. The formulations could be utilized to suppress keloid formation and hypertrophic scar formation by, e.g._, daily application of such a formulation (e.g., for a finite number of days of, e.g._,7 to 28 days) to a surgical scar after surgery (e.g., at the site of incision).

The continuous hydrophobic phase does not include a fatty acid triglyceride. In some embodiments, the continuous hydrophobic phase includes from about 35%, 40%, 50%, 60% or 70 % to about 70%, 80%, 90% or 95% of the total formulation.

A formulation comprises a continuous hydrophobic phase, a hydrophilic phase, at least one hydrophilic compound substantially dissolved in the hydrophilic phase, and a stabilizing component. The continuous hydrophobic phase is a hydrophobic serum composed of cyclopentasiloxane , squalane, dimethiconol , ethoxyheptyl bicyclooctanone, ubiquinone, various extracts, tetrahexyldecyl ascorbate, safflower seed oil, Oenorhera Biennis (evening primrose) oil, tocopherol linoleate/oleate, octyldodecyl citrate crosspolymer, tocopherol acetate, polydecene, caprylic/capric triglyceride, dimethicone, and corn oil. The stabilizing component comprises TPGS and, optionally, at least one emulsifier having an HLB value of less than 10. The formulation comprises at least about 0.8 mg of the hydrophilic compound(s) per 1 g of the continuous hydrophobic phase.

A topical formulation comprises or consists of a continuous hydrophobic phase, a hydrophilic phase, a stabilizing component, and at least about 0. 1% gig of epigallocatechin gallate (EGCG) compound per the continuous hydrophobic phase. The stabilizing component comprises at least one polysorbate emulsifier having an HLB value of at least 10 and, optionally, at least one emulsifier having an HLB value of less than 10. The continuous hydrophobic phase is composed of cyclopentasiloxane, squalane, dimethiconol, ethoxyheptyl bicyclooctanone (ETHOCYN^{®}), ubiquinone, vanous extracts, tetrahexyldecyl ascorbate, safflower seed oil, Oenoz'hem bienm's (evening primrose) oil, tocopherol linoleate/oleate, octyldodecyl citrate crosspolymer, tocopherol acetate, polydecene, caprylic/capric triglyceride, dimethicone, and corn oil. The topical formulation may be stable at room temperature, without agitation, for at least one week.

A topical formulation comprises a continuous hydrophobic phase, a hydrophilic phase, a stabilizing component, and at least about 0.1% (preferably at least about 1% gig) of epigallocatechin gallate (EGCG) compound per the continuous hydrophobic phase. The stabilizing component comprises TPGS and, optionally, at least one emulsifier having an HLB value of less than 10. The continuous hydrophobic phase is composed of cyclopentasiloxane, squalane, dimethiconol , ethoxyheptyl bicyclooctanone (ETHOCYN (Iii), ubiquinone, various extracts, tetrahexyldecyl ascorbate, safflower seed oil, Oenoz`hem Biennis (evening primrose) oil, tocopherol linoleate/oleate, octyldodecyl citrate crosspolymer, tocopherol acetate, polydecene , caprylic/capric triglyceride , dimethicone, and corn oil. The topical formulation may be stable at room temperature , without agitation, for at least one week.

A topical formulation comprises or consists of a continuous hydrophobic phase comprising squalane, a hydrophilic phase, a stabilizing component, and at least about 1% gig of epigallocatechin gallate (EGCG) compound per the continuous hydrophobic phase. The stabilizing component comprises at least one polysorbate emulsifier having an HLB value of at least 10, and at least one emulsifier having an HLB value of less than 10. The topical formulation may be in the form of nanoemulsion and stable at room temperature, without agitation, for at least one week.

A topical formulation comprises or consists of a continuous hydrophobic phase, a hydrophilic phase comprising polyethylene glycol (PEG), a stabilizing component, and at least about 1% gig of green tea extract per the continuous hydrophobic phase. The continuous hydrophobic phase comprises mineral, squalane, or a mixture thereof. The amount of hydrophilic phase in the formulation is at least about 1% gig the hydrophilic phase per the continuous hydrophobic phase. The stabilizing component comprises at least one polysorbate emulsifier having an HLB value of at least 10, and at least one emulsifier having an HLB value of less than 10.

A formulation may comprises or consists of a continuous hydrophobic phase, a hydrophilic phase, at least one hydrophilic compound substantially dissolved in the hydrophilic phase, and a stabilizing component comprising at least one polysorbate emulsifier having an HLB value of at least 10, and at least one emulsifier having an HLB value ofless than 10. About 8% to about 30% by weight of the stabilizing component is polysorbate emulsi:fler(s) having an HLB value of at least 10. The formulation comprises no more than about 4% by weight of the polysorbate emulsifier(s) having an HLB value of at least 10. The continuous hydrophobic phase may comprise mineral oil (e.g. DRAKEOL 35 from Penreco), vegetable oil, paraffin, squalene, hemisqualane or a mixture thereof. The hydrophilic phase may comprise water, polyethylene glycol, propylene glycol, or a mixture thereof. The weight ratio of the hydrophilic compound(s) to the hydrophilic phase may be from about 1:5 to about 1:10. The weight ratio of the continuous hydrophobic phase to the hydrophilic phase is at least 10:1. The weight ratio of the stabilizing 17 component to the continuous hydrophobic phase is from about 1:1 to about **1:10.** The formulation may comprise at least about 10 mg of the hydrophilic compound(s) per 1 g of the continuous hydrophobic phase.

About 7.5% to about 12% by weight of the stabilizing component may be the polysorbate emulsifier(s) having an HLB value of at least 10.

A formulation comprises or consists of a continuous hydrophobic phase comprising mineral oil, a hydrophilic phase comprising polyethylene glycol, at least one hydrophilic compound substantially dissolved in the hydrophilic phase, and a stabilizing component comprising at least one polysorbate emulsifier having an HLB value of at least 10, and at least one emulsifier having an HLB value of less than 10. About 10% by weight of the stabilizing component is the polysorbate emulsifier(s) having an HLB value of at least 10. The weight ratio of the hydrophilic compound(s) to the hydrophilic phase is about 1:10. The weight ratio of the hydrophilic phase to the continuous hydrophobic phase is about 1:10. The weight ratio of the stabilizing component to the continuous hydrophobic phase is about 1:1. The formulation comprises at least about 10 mg of the hydrophilic compound(s) per 1 g of the continuous hydrophobic phase.

A formulation comprises or consists of a continuous hydrophobic phase comprising squalane, a hydrophilic phase comprising propylene glycol, at least one hydrophilic compound substantially dissolved in the hydrophilic phase, and a stabilizing component comprising at least one polysorbate emulsifier having an HLB value of at least 10, and at least one emulsifier having an HLB value of less than 10 and. About 8% to about 30% by weight of the stabilizing component is polysorbate emulsifier(s) having an HLB value of at least 10. The formulation comprises no more than about 4% by weight of the polysorbate emulsifler(s) having an HLB value of at least 10. The weight ratio of the hydrophilic compound(s) to the hydrophilic phase is about 1:5. The weight ratio of the hydrophilic phase to the continuous hydrophobic phase is about 1:10. The weight ratio of the stabilizing component to the continuous hydrophobic phase is from about 1:4 to about 1:10. The formulation comprises at least about 20 mg of the hydrophilic compound(s) per 1 g of the continuous hydrophobic phase.

A topical formulation compnses or consists of a continuous squalane hydrophobic phase, a propylene glycol hydrophilic phase, an epigallocatechin gallate (EGCG) compound in an amount from about 1.0% to about 1.7% by weight based on total weight of the topical formulation, and a stabilizing component. The weight ratio of the continuous squalane hydrophobic phase to the propylene glycol hydrophilic phase is at least about 10:1. The stabilizing component comprises at least one polysorbate surfactant(s) having an HLB value of at least 10, and at least one fatty acid monoester of sorbitan. The polysorbate surfactant(s) comprises from about 8% to about 30% by weight based on total weight of the stabilizing component, and no more than about **3.5%** by weight based on total weight of the topical formulation.

About **7.5%** to about 12% by weight of the stabilizing component may be the polysorbate emulsifier(s) having an HLB value of at least 10.

A formulation comprises or consists of a continuous hydrophobic phase comprising mineral oil, a hydrophilic phase comprising polyethylene glycol, at least one hydrophilic compound substantially dissolved in the hydrophilic phase, and a stabilizing component comprising at least one polysorbate emulsifier having an HLB value of at least 10, and at least one emulsifier having an HLB value of less than 10. About 10% by weight of the stabilizing component is the polysorbate emulsifier(s) having an HLB value of at least 10. The weight ratio of the hydrophilic compound(s) to the hydrophilic phase is about **1:10.** The weight ratio of the hydrophilic phase to the continuous hydrophobic phase is about **1:10.** The weight ratio of the stabilizing component to the continuous hydrophobic phase is about **1:1.** The formulation comprises at least about 10 mg of the hydrophilic compound(s) per **1** g of the continuous hydrophobic phase.

A formulation comprises or consists of a continuous hydrophobic phase comprising squalane, a hydrophilic phase comprising propylene glycol, at least one hydrophilic compound substantially dissolved in the hydrophilic phase, and a stabilizing component comprising at least one polysorbate emulsifier having an HLB value of at least 10, and at least one emulsifier having an HLB value of less than 10 and. About 8% to about 30% by weight of the stabilizing component is polysorbate emulsifier(s) having an HLB value of at least 10. The formulation comprises no more than about 4% by weight of the polysorbate emulsifier(s) having an HLB value of at least 10. The weight ratio of the hydrophilic compound(s) to the hydrophilic phase is about **1:5.** The weight ratio of the hydrophilic phase to the continuous hydrophobic phase is about **1:10.** The weight ratio of the stabilizing component to the continuous hydrophobic phase is from about 1:4 to about **1:10.** The formulation comprises at least about 20 mg of the hydrophilic compound(s) per **1** g of the continuous hydrophobic phase.

A topical formulation comprises or consists of a continuous squalane hydrophobic phase, a propylene glycol hydrophilic phase, an epigallocatechin gallate (EGCG) compound in an amount from about **1.0%** to about **1.7%** by weight based on total weight of the topical formulation, and a stabilizing component. The weight ratio of the continuous squalane hydrophobic phase to the propylene glycol hydrophilic phase is at least about **10:1.** The stabilizing component comprises at least one polysorbate surfactant(s) having an HLB value of at least 10, and at least one fatty acid monoester of sorbitan. The polysorbate surfactant(s) comprises from about 8% to about 30% by weight based on total weight of the stabilizing component, and no more than about **3.5%** by weight based on total weight of the topical formulation.

IA topical formulation comprises or consists of a continuous hydrophobic phase, a hydrophilic phase comprising propylene glycol, at least one hydrophilic compound substantially dissolved in the hydrophilic phase, and a stabilizing component comprising at least one polysorbate emulsifier having an HLB value of at least 10. The weight ratio of the hydrophilic compound(s) to the hydrophilic phase is about **1:5.** The weight ratio of the hydrophilic phase to the continuous hydrophobic phase is about **1:240.** The weight ratio of the stabilizing component to the continuous hydrophobic phase is about **121000.** The topical formulation comprises at least about 0.8 mg of the hydrophilic compound(s) per 1 g of the continuous hydrophobic phase. The continuous hydrophobic phase comprises cyclopentasiloxane, squalane, dimethiconol, ethoxyheptyl bicyclooctanone (ETHOCYN^{®}), ubiquinone, various extracts, tetrahexyldecyl ascorbate, safflower seed oil, Oenoz`hem Biennis (evening primrose) oil, tocopherol linoleate/oleate, octyldodecyl citrate crosspolymer, tocopherol acetate, polydecene, caprylic/capric triglyceride, dimethicone, and corn oil.

The formulation does not include a fatty acid triglyceride.

The foregoing formulation(s) may further include a lower alkyl-substituted bicyclo[3.3.0]octan-3-one compound, such as 6-(5 -ethoxyhept-1-yl)bicyclo[3.3.0]octan-3-one (available under the trade name ETHOCYN^{®} from BCS Business Consulting Services Pte Ltd of Singapore) or 6-(5-methoxyhept-1-yl)bicyclo[3 .3.0]octan-3-one. Methods of manufacture, and uses of skin care compositions comprising a lower alkyl-substituted bicyclo[3 .3.0]octan-3-one compound such as 6-(5-ethoxyhept-1-yl)bicyclo[3 .3.0]octan-3-one ("ETHOCYN^{®}") or 6-(5-methoxyhept-1-yl)bicyclo[3.3.0]octan-3-one (CYOCTOLTM) are described in detail in US. Patents 4,689,349, 4,689,345, and 4,855, 22.

The formulation(s) may further include an additional ingredient, a pharmaceutically and/or cosmetically acceptable additive, or both. As non-limiting examples, the formulations may further include at least one of the following: adjuvants, excipients, solubilizers, thickeners, gelling agents, fillers, colorants, lubricants, binders, moisturizing agents, preservatives, fragrances, electrolytes, adsorption enhancers, skin penetration enhancers, UV blocking materials; antioxidants, neutralizing agents, buffering agents, and viscosity enhancers (e.g., bee's wax).

The formulations may be in various forms, e.g., as a gel, ointment, or dermal adhesive patch.

The formulations may be prepared according to known methods for dispersing a hydrophilic phase in a continuous hydrophobic phase.

A hydrophilic compound is combined with a hydrophilic solvent to provide a solution of the hydrophilic compound. A selected stabilizing component is added to the solution of the hydrophilic compound. Upon combining with a hydrophobic carrier, a stabilized formulation is obtained that comprises a continuous hydrophobic phase and a dispersed phase in the form of reverse micelles that include a hydrophilic component.

The method of preparing a formulation comprises dissolving at least one hydrophilic compound in a hydrophilic phase to provide a solution of the hydrophilic compound(s), and combining the solution with a continuous hydrophobic phase to provide the formulation. The method further comprises adding a stabilizing component that include at least one polysorbate emulsifier having an HLB value of at least 10 and at least one emulsifier having an HLB of less than 10. About 8% to about 30% by weight of the stabilizing component is polysorbate emulsifier(s) having an HLB value of at least 10. The formulation comprises no more than about 4% by weight of the polysorbate emulsifier(s) having an HLB value of at least 10.

The method of preparing a formulation may comprise dissolving at least one hydrophilic compound in a hydrophilic phase to provide a solution of at least one hydrophilic compound, and then adding a stabilizing component to the solution to produce a stabilizing component-containing solution of the hydrophilic compound(s). The method further comprises combining the stabilizing component-containing solution with a continuous hydrophobic phase to provide the formulation. The stabilizing component includes at least one polysorbate emulsifier having an HLB value of at least 10 and at least one emulsifier having an HLB ofless than 10, wherein from about 8% to about 30% by weight of the stabilizing component is polysorbate emulsifier(s) having an HLB value of at least 10. The formulation comprises no more than about 4% by weight of the polysorbate emulsifier(s) having an HLB value of at least 10.

Preferred formulations display enhanced shelf life, and tolerate a relatively wide range of temperatures and other conditions. Such formulations are stable at room temperature, without agitation, for at least one week. The formulations are stable for at least about 3 years.

A topical formulation comprises a continuous hydrophobic phase, a hydrophilic phase, at least about 0.1% (preferably at least about 1%) by weight of green tea extract, e.g., epigallocatechin gallate (EGCG) compound based upon total weight of the topical formulation, and a stabilizing component in an amount of at least about 15% by weight based upon total weight of the topical formulation. The continuous hydrophobic phase comprises mineral oil, squalane, or a mixture thereof. The stabilizing component comprises TPGS in an amount from about 8% to about 30% by weight, preferably from about **7.5%** to about 12% by weight, based upon total weight of the stabilizing component, and at least one emulsifier having an HLB value of less than **10** (preferably about 3 to 8, and more preferably about 3 to 6). A weight ratio of the stabilizing component to the hydrophilic compound(s) is at least about **10:1.**

A topical formulation comprises a continuous squalane hydrophobic phase, a propylene glycol hydrophilic phase, an EGCG compound in an amount from about **1.0%** to about **1.7%** by weight based upon total weight of the topical formulation , and a stabilizing component in an amount of at least about 15 % by weight based upon total weight of the formulation. The stabilizing component comprises TPGS in an amount from about 8% to about 30% by weight based upon total weight of the stabilizing component and at least one fatty acid monoester of sorbitan having an HLB value of less than 6. A weight ratio of the continuous squalane hydrophobic phase to the propylene glycol hydrophilic phase is about **10:1. A** weight ratio of the continuous squalane hydrophobic phase to the stabilizing component is no more than about **5:1. A** weight ratio of the stabilizing component to the EGCG compound is at least about **10:1.**

In further particular embodiments, the formulations do not include triglyceride.

The topical formulations further include ETHOCYN^{®} (from BCS Business Consulting Services Pte Ltd of Singapore) or 6-(5-methoxyhept-1-yl)bicyclo[3.3.0]octan-3-one (CYOCTOLTM). Methods of manufacture, and uses of skin care compositions comprising a 4,689,349, 4,689,345, and 4,855, 22.

The formulations further include an additional ingredient, a pharmaceutically and/or cosmetically acceptable additive, or both. As non-limiting examples, the formulations may further include at least one of the following: an adjuvant, an excipient, a solubilizer, a thickener, a gelling agent, a filler, a colorant , a lubricant, a binder, a moisturizing agent, a preservative, a fragrance, an adsorption enhancer, an UV blocking material; an antioxidant , a neutralizing agent, a buffering agent, and s viscosity enhancer (e.g, bee's wax).

The formulations may be in various forms, e.g., as a gel, ointment, or dermal adhesive patch.

The formulations may be prepared according to known methods for dispersing a hydrophilic phase in a continuous hydrophobic phase.

In some embodiments, the formulations may be prepared by dissolving at least one hydrophilic compound in a hydrophilic phase to provide a solution of the hydrophilic compound(s). Then, TPGS may be added to the solution of the hydrophilic compound(s). Upon combining the solution with a mixture comprising a hydrophobic carrier and, optionally, at least one emulsifier having a HLB value of less than 10, a stabilized formulation may be obtained that comprise a continuous hydrophobic phase and a dispersed phase in the form of reverse micelles that include the hydrophilic component(s).

The formulations may have enhanced shelf stability or shelf life, and tolerate a wide range of temperatures and other conditions. The formulations may be stable at room temperature, without agitation, for at least one week. The formulations may be stable for at least about 3 years.

The formulations may be administered to a subject topically, such as in the form of ointments, drops or transdermal patches, as known in the pharmaceutical art or cosmetic art.

The formulations may be used to treat a condition (ag, to cure an abnormal condition, to prevent an undesirable condition, and/or to enhance a desirable condition) in a mammal, preferably human, in need thereof. The method of treating such a condition may involve topically administering the formulations to the subject in need of treatment. Accordingly, a method for treating a condition in a subject in need thereof comprises topically administering the formulation to the subject.

The formulations may be useful for delivering a hydrophilic compound to any animal, but preferably mammals. Other uses are contemplated, for example application of a compound to an inanimate surface.

Also disclosed is a method for delivering a hydrophilic compound to a subject.
The method comprises, for example, topically administering the formulation to the subject.

Upon a topical application of the formulation to a skin, at least a portion of the hydrophilic compound contained in the reverse micelles may leave or disassociate from the reverse micelle and then transport through the skin. Thus, layer under the skin may benefit from the effect of the hydrophilic compound.

Upon a topical application of the formulation to a skin, at least a portion of the hydrophilic compound contained in the reverse micelles may transport through the skin and then leave or disassociate from the reverse micelle. Thus, a systemic delivery of the hydrophilic compound to the layer under skin may be achieved.

The disclosure is further described with the aid of the following illustrative Examples.

### EXAMPLES

### EXAMPLE I: Formulations Comprising Hydrophilic EGCG Compound in a Continuous Hydrophobic Phase With or Without TPGS Emulsifier

The hydrophilic epigallocatechin gallate (EGCG, (catechins (tea extract) SunphenonTM EGCg from Taiyo Green Power Co., Ltd of Wuxi Jiangsu, China -- distributed as Stauber Item# 24845 by Stauber of California)) compound was extracted from green tea. Propylene glycol (PG) was used as the hydrophilic solvent. Squalane (OleaClearTM Olive Squalane from Tri-K Industries of Northvale , NJ, US) and mineral oil hydrophobic solvents were tested as the continuous hydrophobic phase. TPGS emulsifier was obtained from Eastman Chemical Company. Sorbitan monooleate surfactant having an HLB value of 4.3 (SPANTM 80 surfactant from Croda hie.) was used as the emulsifier having an HLB value ofless than 10.

The EGCG compound was dissolved in propylene glycol (PG) hydrophilic solvent to produce a solution (in, hydrophilic phase) having a weight ratio of EGCG compound to PG solvent of about 1:5. The solution of EGCG was mixed with a predetermined amount of TPGS emulsifier, and then combined with a mixture of the selected hydrophobic solvent and sorbitan monooleate surfactant at a weight ratio of the hydrophilic solvent PG to the hydrophobic solvent of about 1:10.

To test the effect of the stabilizing component on the stability of the formulation, the formulations having various combinations and amounts of the stabilizing component were prepared as shown in TABLE 1. The formulations were tested for stability at room temperature.

As shown in TABLE 1, Formulations A1, B1, C1, D1 and E1 were not stable when the stabilizing component in the formulations did not include any TPGS emulsifier, regardless the amounts of sorbitan monooleate. Thus, the formulations required TPGS emulsifier to enhance their shelf stabilities, and sorbitan monooleate surfactant (HLB value of 4.3) alone was not sufficient. The formulations using squalane hydrophobic solvent showed similar stability as those using mineral oil as a hydrophobic solvent. The stability of the formulations decreased as the weight ratio of the stabilizing component to the EGCG compound reduced. Formulations E2, E3, and EX showed that the weight ratio of the stabilizing component to the EGCG compound of at least about 10:1 was required for an enhanced stability of the formulations.

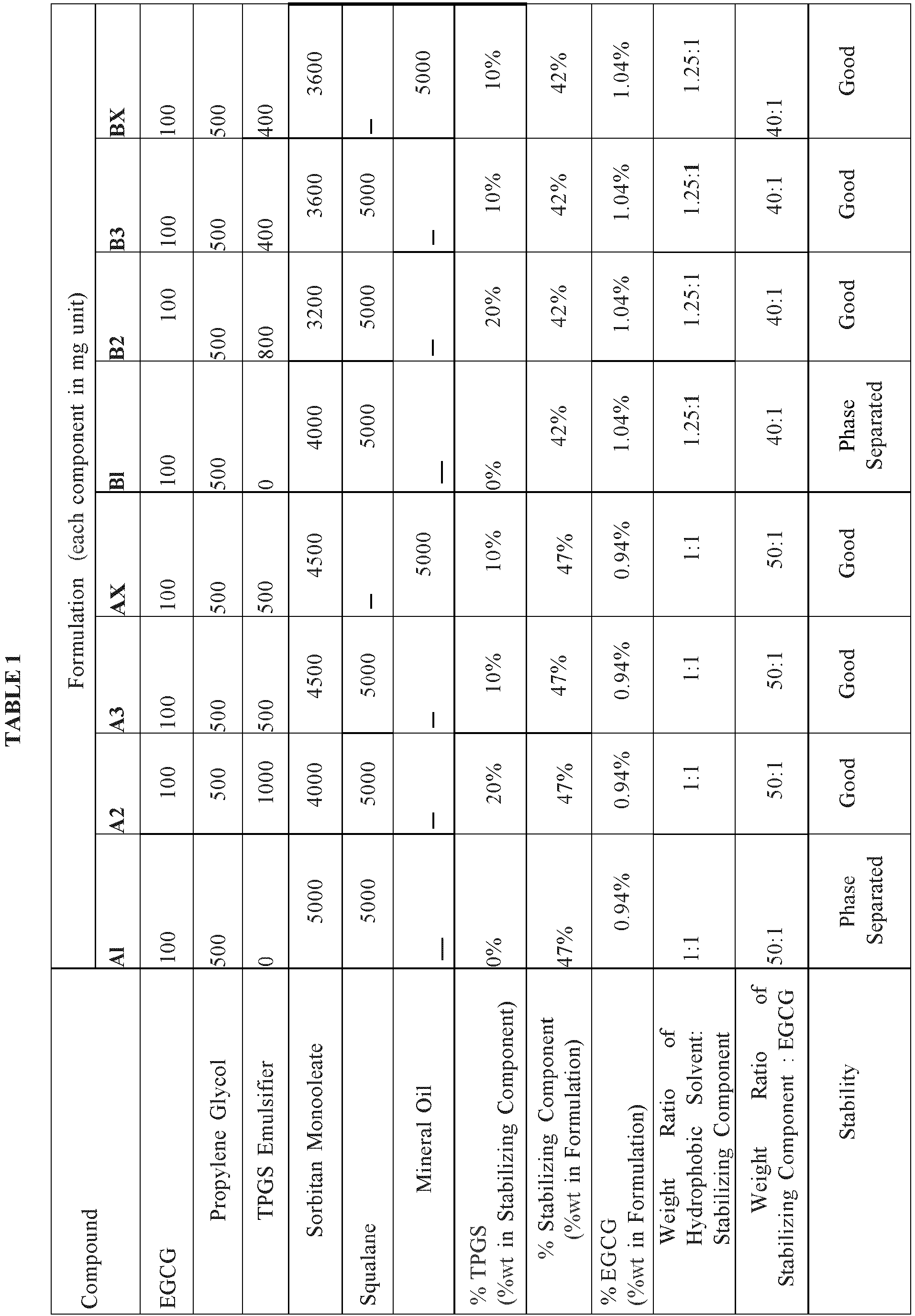

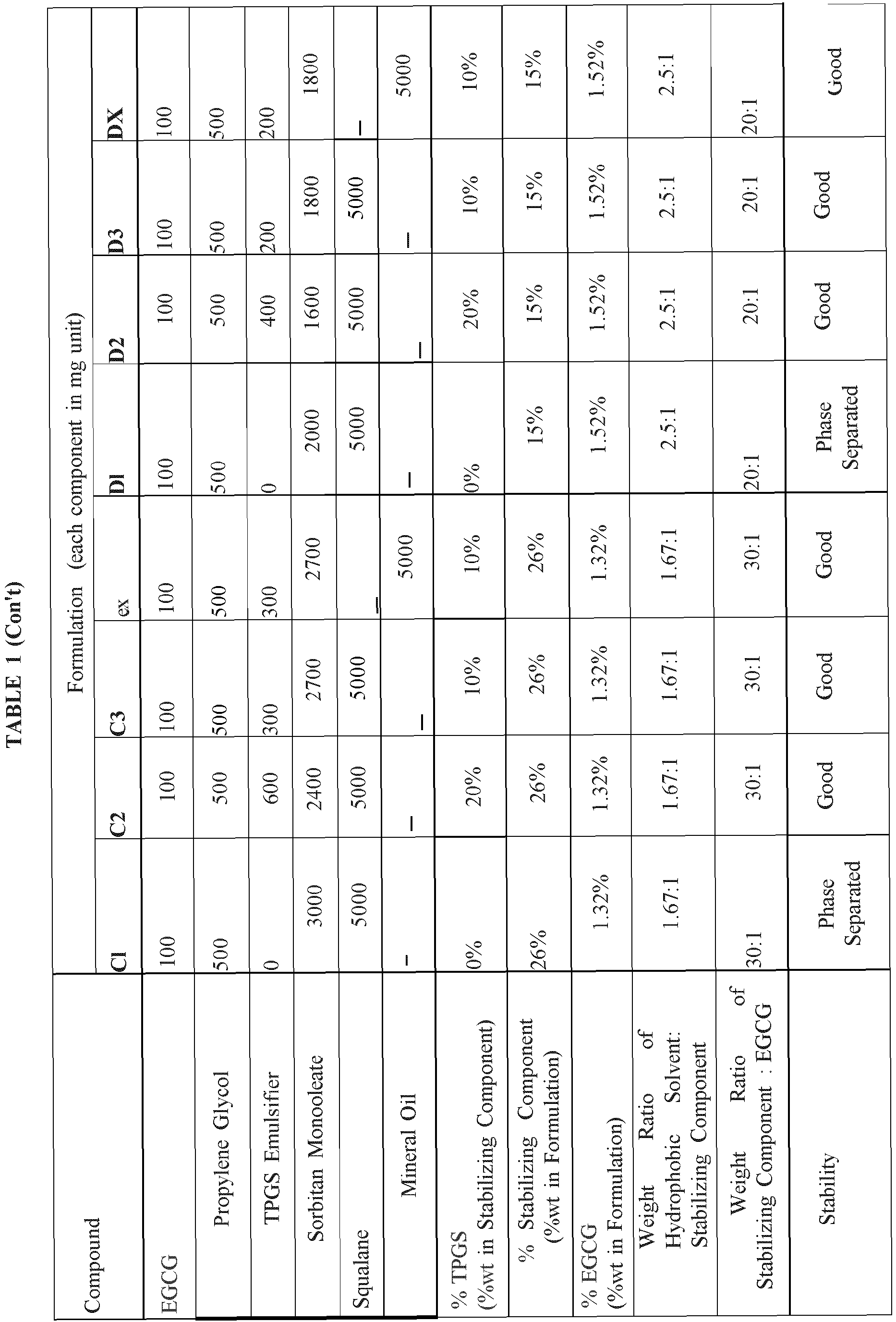

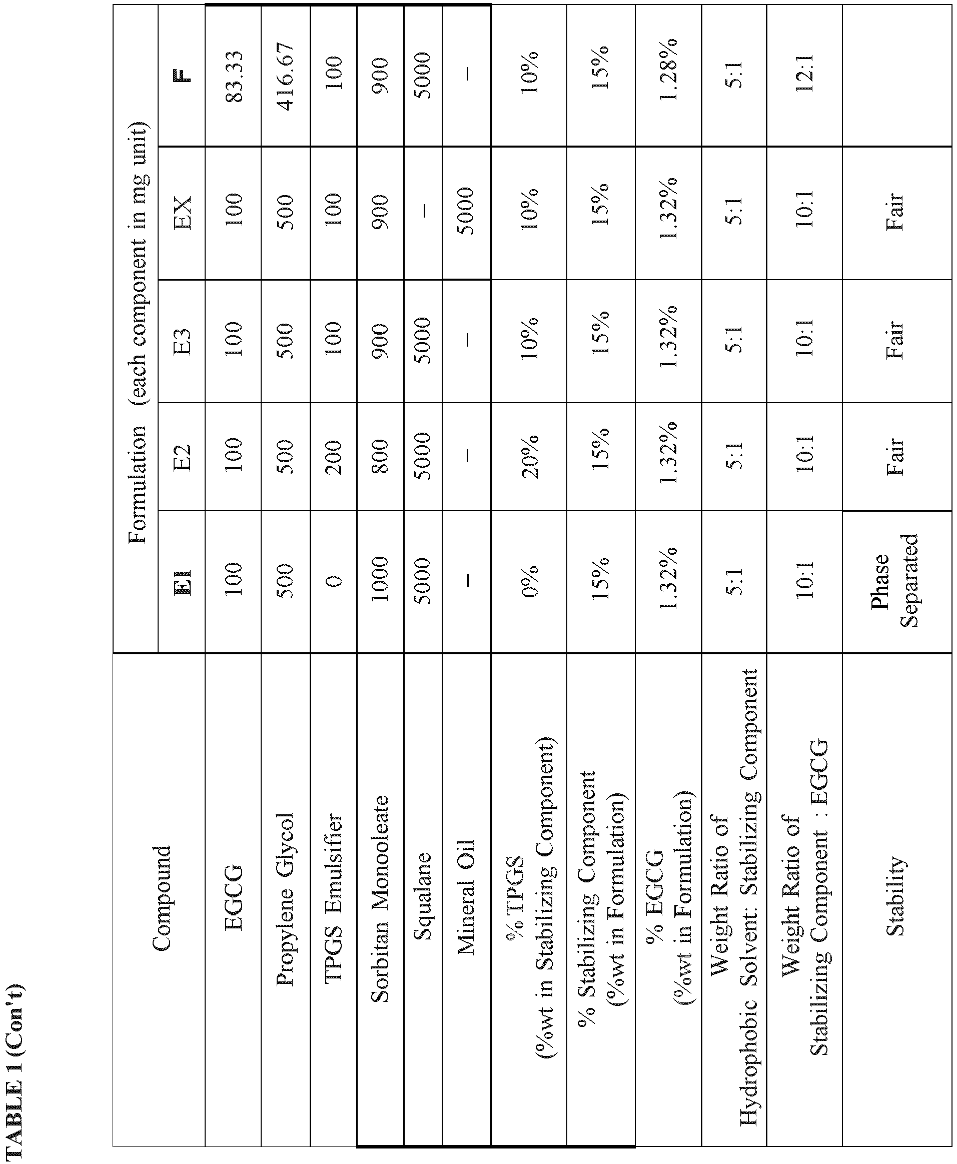

### EXAMPLE 2: Formulation F Comprising Hydrophilic EGCG Compound in a Hydrophobic Squalane Solvent with a Stabilizing Component Comprising TPGS and Sorbitan Monooleate Emulsifiers

About 4 g of a hydrophilic EGCG compound was dissolved in about 20 g of a propylene glycol hydrophilic solvent, and the mixture was heated to about 60°C to provide a solution (to, hydrophilic phase) having a weight ratio of the hydrophilic EGCG compound to the propylene glycol hydrophilic solvent of about **1:5.** To about 500 mg of the solution, about **100** mg of TPGS emulsifier was added. The resulting solution was then combined with a mixture composed of **5000** g squalane hydrophobic solvent and 900 mg of sorbitan monooleate surfactant (SPANTM 80 surfactant, HLB value of 4.3) and stirred vigorously to provide Formulation **F** of **TABLE 1.** Formulation F showed good stability at room temperature for at least one week without agitation. See **TABLE 1,** supra.

### EXAMPLE 3: Formulations Comprising Hydrophilic EGCG Compound in a Hydrophobic Squalane Solvent Using Different Stabilizing Components

The EGCG compound was dissolved in propylene glycol (PG) hydrophilic solvent to produce a solution (216., hydrophilic phase) having a weight ratio of EGCG compound to PG solvent of about **1:5.** The solution of EGCG was mixed with a predetermined amount of TPGS emulsifier, and then combined with a mixture composed of squalane hydrophobic solvent and sorbitan monooleate surfactant at a weight ratio of the hydrophilic solvent PG to the hydrophobic squalane solvent of about **1:10.** To test the effect of the stabilizing component on the stability of the formulation, the formulations having various combinations and amounts of the stabilizing component were prepared as shown in **TABLE** 2. The formulations were tested for stability at room temperature.

As shown in **TABLE** 2, when squalane was used as the continuous phase for the formulation of EGCG hydrophilic compound, the stability of the formulation was achieved by using a stabilizing component composed of both TPGS emulsifier and an emulsifier having an HLB value less than **10** (ag, sorbitan monooleate, HLB value of 4.3). The formulations were not stable when the amounts of the stabilizing component failed below 15 % by weight-based upon total weight of the formulations. Furthermore, the stability of the formulations decreased as the weight ratio of the stabilizing component to the EGCG compound reduced to below **10:1.**

**TABLE 2**

| Compound | Formulation (each component in mg unit) | | | | | | | | | | | |
|---|---|---|---|---|---|---|---|---|---|---|---|---|
| | **I** | **II** | **III** | **IV** | **V** | **VI** | **VII** | **VIII** | **IX** | **X** | **XI** | **XII** |
| EGCG | 100 | 100 | 100 | 100 | 100 | 100 | 100 | 100 | 100 | 100 | 100 | 100 |
| Propylene glycol | 500 | 500 | 500 | 500 | 500 | 500 | 500 | 500 | 500 | 500 | 500 | 500 |
| TPGS Emulsifier | 200 | 200 | 200 | 200 | 200 | 200 | 100 | 100 | 100 | 100 | 100 | 100 |
| Sorbitan Monoolea | 500 | 600 | 700 | 800 | 900 | 1000 | 500 | 600 | 700 | 800 | 900 | 1000 |
| Squalane | 5000 | 5000 | 5000 | 5000 | 5000 | 5000 | 5000 | 5000 | 5000 | 5000 | 5000 | 5000 |
| TPGS (%wt in Stabilizing | 29% | 25% | 22% | 20% | 18% | 17% | 17% | 14% | 13% | 11% | 10% | 9% |
| Stabilizing Component (%wt in Form.) | 11% | 13% | 14% | 15% | 16% | 18% | 10% | 11% | 13% | 14% | 15% | 16% |
| EGCG (%wt in Form.) | 1.59% | 1.56% | 1.54% | 1.52% | 1.49% | 1.47% | 1.61% | 1.59% | 1.56% | 1.54% | 1.52% | 1.49% |
| Weight Ratio of Hydrophobic Solvent: Stabilizing | 7.14:1 | 6.25:1 | 5.56:1 | 5:1 | 4.55:1 | 4.17:1 | 8.33:1 | 7.14:1 | 6.25: 1 | 5.56:1 | 5:1 | 4.55:1 |
| Weight Ratio of Stabilizing | 7:1 | 8:1 | 9: 1 | 10:1 | 11:1 | 12:1 | 6:1 | 7:1 | 8:1 | 9:1 | 10:1 | 11:1 |
| Stability | Phase Separate d | Phase Separate d | Phase Fair Separate d | Good | Good | Phase Separate d | Phase Separate d | Phase Separate d | Fair | Good | | Good |

### EXAMPLE 4: Formulation Comprising Hydrophilic EGCG Compound in a Hydrophobic Squalane Solvent

About 4 grams of the hydrophilic EGCG compound was mixed with about 20 grams of propylene glycol (hydrophilic solvent). The mixture was heated to about 60°C until the hydrophilic EGCG compound was fiilly dissolved in the hydrophilic solvent to provide a EGCG solution (219., hydrophilic phase) having a weight ratio of the hydrophilic EGCG compound to the hydrophilic solvent of about **1:5.**

To a stainless steel mortar and pestle, about 5 grams of squalane (or, alternatively, an equal amount of medicinal liquid paraffin) was added and heated to about 60°. Then, about 0.9 grams of sorbitan monooleate surfactant having an HLB value of 4.3 (SPANTM 80 surfactant from Croda Inc.) was added and mixed well with the squalane. About 500 mg of the EGCG solution (ta, hydrophilic phase) and about 100 mg of TPGS emulsifier were then added to the stainless steel mortar and pestle, and the mixture in the stainless steel mortar and pestle was mixed immediately. Then, the resulting mixture was allowed to cool down to room temperature under continued mixing to provide a formulation comprising about **1.3%** by weight of the hydrophilic EGCG compound based on total weight of the formulation. After that, a portion of the formulation was transferred from the stainless steel mortar and pestle to a clear vial for a stability study. The formulation was a clear liquid having a light orange-beige color without opalescence or haze. The formulation showed an improved stability.

EXAMPLE 5: Formulation Comprising Hydrophilic EGCG Compound in Anhydrous Hydrophobic Serum

An anhydrous hydrophobic serum was prepared composed of cyclopentasiloxane, squalane (OleaClearTM Olive Squalane from Tri-K Industries of Northvale, NJ, US), dimethiconol, ethoxyheptyl bicyclooctanone (ETHOCYN^{®}), ubiquinone, various extracts, tetrahexyldecyl ascorbate, safflower seed oil, Oenoz`hem Biennis (evening primrose) oil, tocopherol linoleate/oleate, octyldodecyl citrate crosspolymer, tocopherol acetate, polydecene, caprylic/capric triglyceride, dimethicone, and corn oil.

The hydrophilic epigallocatechin gallate (EGCG) compound is readily commercially available (e.g., "catechins" (tea extract) SunphenonTM EGCg from Taiyo Green Power Co., Ltd of Wuxi Jiangsu, China -distributed as Stauber Item# **24845** by Stauber of California, US).

Various formulations compnsmg the hydrophilic EGCG compound in the anhydrous hydrophobic serum at different concentrations were prepared using Methods 1, 2, or 3. The resulting formulations were tested for stability at room temperature.

### Method 1: Direct dispersion of hydrophilic EGCG compound into the anhydrous hydrophobic serum

Hydrophilic EGCG compound from green tea extract was mixed directly with the anhydrous hydrophobic serum at various concentrations with or without colloidal silicon dioxide: **0.1%, 0.2%, 0.5%,** and 10% by weight of EGCG compound per total weight of the formulation.

The produced formulations at all tested EGCG concentrations were stable at room temperature without agitation for only a few hours, and then precipitation and/or creaming of the formulations were observed.

Method 2: Dispersion of a hydrophilic solution of hydrophilic EGCG compound into the anhydrous hydrophobic serum without using any stabilizing component **[00100]** The hydrophilic EGCG compound was dissolved in a macrogol polyethylene glycol (PEG **800)** hydrophilic solvent to produce a solution (116., hydrophilic phase) having a weight ratio of the hydrophilic EGCG compound to the PEG 800 hydrophilic solvent of about **1:10.** The hydrophilic phase was then mixed with the anhydrous hydrophobic serum at various concentrations to provide the formulations containing about **50** to **1000** mL of the PEG 800 hydrophilic phase per **5** mL of the anhydrous hydrophobic serum.

The tests showed that up to about 10% gig of the hydrophilic EGCG compound per the anhydrous hydrophobic serum could be produced without losing integrity of the anhydrous hydrophobic serum. However, such formulations had poor stability and phase separated within one day at room temperature without agitation.

Method 3: Dispersion of a hydrophilic solution of hydrophilic EGCG compound into the anhydrous hydrophobic serum using a stabilizing component

About 4 g of a hydrophilic EGCG compound was dissolved in about **20** g of a propylene glycol hydrophilic solvent, and the mixture was heated to about **60°C** to provide a solution (116., hydrophilic phase) having a weight ratio of the hydrophilic EGCG compound to the propylene glycol hydrophilic solvent of about **1:5.** To about 500 mg of the solution, about 100 mg of polysorbate **80** surfactant having an HLB value of **15** (PEG-20 sorbitan monooleate, TWEEN^{®} **80** from Croda Inc.) was added. Then, the hydrophobic serum was gradually added while the mixture was stirred vigorously until the total weight of the mixture/formulation was 100g.

The resulting formulation had a weight ratio of the hydrophilic phase to the continuous hydrophobic phase is about **1:240,** and a weight ratio of the stabilizing component to the continuous hydrophobic phase is about **1:1000.** The formulation was composed of about **0.8** mg of the active EGCG compound per **1** g of the hydrophobic serum. The formulation showed excellent stability for up to 3 weeks at room temperature without agitation.
- - Therefore by using a stabilizing component, the-stable formulation composed of about **0.8** mg of the active EGCG compound per 1 g of the hydrophobic serum was achieved.

### EXAMPLE 6: Formulation Comprising Hydrophilic EGECG Compound in a Hydrophobic Mineral Oil Solvent

The hydrophilic EGCG compound was dissolved in a macrogol polyethylene glycol PEG 800 (hydrophilic solvent) to produce a solution **(1.6.,** hydrophilic phase) having a weight ratio of the hydrophilic EGCG compound to the hydrophilic solvent of about **1:10.** Then, the hydrophilic phase was mixed with mineral oil (hydrophobic solvent) to provide a formulation having a weight ratio of the hydrophilic solvent (macrogol PEG 800) to the mineral oil of about **1:10.**

To test the effect of the stabilizing component on the stability of the formulation, the hydrophilic phase was mixed with mineral oil using different combinations and amounts of the stabilizing component. Polysorbate 80 surfactant having an HLB value of 15 (PEG-20 sorbitan monooleate, TWEEN^{®} 80 surfactant), and sorbitan monooleate surfactant having an HLB value of 4.3 (SPANTM 80 surfactant) were studied as the stabilizing component.

Various combinations of polysorbate 80 and sorbitan monooleate emulsifiers were tested as the stabilizing component for the formulation of active EGCG compound, and at various weight amount ratios in relation to the hydrophobic mineral oil, as shown in TABLE 3.

**TABLE3**

| Compound | Formulation (each component in mg unit) | | | | | | | |
|---|---|---|---|---|---|---|---|---|
| | **A** | B | c | D | F | H | I | |
| EGCG hydrophilic cpd | 50 | 50 | 50 | 50 | 50 | 50 | 50 | |
| PEG800 | | | | | | | | |
| | *Hydrophilic solvent* | *500* | *500* | *500* | *500* | *500* | *500* | *500* |
| | Mineral *Oil Hydrophobic solvem'* | 5000 | 5000 | 5000 | 5000 | 5000 | 5000 | 5000 |
| | *Polysorbate* | | | | | | | |
| | *High HLB emulsifier* | 0 | *500* | *500* | *500* | *500* | 0 | 0 |
| | *Sorbitan Monooleate* | | | | | | | |
| | *Low HLB emulsifier* | 0 | 0 | *500* | 1000 | 4500 | 2000 | 1000 |
| | *Stability* | *Phase Separated* | *Phase Separated* | *Phase Separated* | *Phase Separated* | *Good* | *Phase Separated* | *Phase Separated* |

*As shown in* TABLE *3, the formulation* **A** *which did not include a stabilizing component showed poor stability (i.e., phase separation). The formulation B, which used only polysorbate emulsifier also showed poor stability. Likewise, the formulations* H *and I, each of which used only the low HLB surfactant, showed poor stability.*

*The most enhanced stability was observed in the formulation F, which included both polysorbate* 80 (HLB *value of 15) and sorbitan monooleate* (HLB *value of 4.3) emulsifiers as the stabilizing component. Formulation* F *had a weight ratio of the stabilizing component to the mineral oil of about 1:1, and about 10% weight of the stabilizing component was polysorbate 80.*

*Therefore, when mineral oil was used as the continuous phase for the formulation of* EGCG *hydrophilic compound, the stability of the formulation was achieved by using a stabilizing component composed of both polysorbate emulsifier having an HLB value of at least* 10 *(e.g., polysorbate* 80, *HLB value of 15) and an emulsifier having an HLB value less than* 10 *(e.g., sorbitan monooleate, HLB value of 4.3). The formulation had a weight ratio of the hydrophilic* EGCG *compound to the hydrophilic solvent (macrogol PEG* 800) *of about* 1:10, *a weight ratio of the hydrophilic solvent (macrogol PEG* 800) *to the mineral oil (hydrophobic solvent) of about* 1:10, *a weight ratio of the stabilizing component to the mineral oil of about 1:1, and about* 10% *weight of the stabilizing component was the polysorbate emulsifier having an HLB value of at least* 10 *(e.g, polysorbate* 80, *HLB value of 15).*

### EXAMPLE 7: Formulations Comprising Hyd rophilic EGCG Compound in a Hydrophobic Squalane Solvent Using Different Stabilizing Components

The hydrophilic EGCG compound was dissolved in propylene glycol (hydrophilic solvent) a solution **(1.6.,** hydrophilic phase) having a weight ratio of the hydrophilic EGCG compound to the hydrophilic solvent of about **1:5.** Then, the hydrophilic phase was mixed with squalane (hydrophobic solvent) to provide a formulation having a weight ratio of the hydrophilic solvent (propylene glycol) to the squalane solvent of about **1:10.**

To test the effect of the stabilizing component on the stability of the formulation, the hydrophilic phase was mixed with the squalane solvent using different combinations and amounts of the stabilizing components. Polysorbate 80 surfactant having an HLB value of 15 **(PEG-20** sorbitan monooleate, TWEEN^{®} 80 from Croda Inc.), and sorbitan monooleate surfactant having an HLB value of 4.3 (SPANTM 80 surfactant from Croda Inc.) were studied as the stabilizing component

Various combinations of polysorbate 80 and sorbitan monooleate emulsifiers were tested as the stabilizing component for the formulation of active EGCG compound, and at various weight amount ratios in relation to the hydrophobic squalane solvent, as shown in TABLE 4.

As shown in TABLE 4, when squalane was used as the continuous phase for the formulation of EGCG hydrophilic compound, the stability of the formulation was achieved by using a stabilizing component composed of both polysorbate emulsifier having an HLB value of at least **10** (e.g., polysorbate 80, HLB value of 15) and an emulsifier having an HLB value less than **10** (e.g., sorbitan monooleate, HLB value of **4.3).**

**TABLE 4**

| | | Formulation (eachcomponentinmgunit) | | | | | | | | | | | |
|---|---|---|---|---|---|---|---|---|---|---|---|---|---|
| Compound | | I | II | III | IV | v | VI | VII | VII | IX | x | XI | XII |
| EGCG *Active Compound* | | 100 | 100 | 100 | 100 | 100 | 100 | 100 | 100 | 100 | 100 | 100 | 100 |
| Propyleneglycol *Hydrophilic solvent* | | 500 | 500 | 500 | 500 | 500 | 500 | 500 | 500 | 500 | 500 | 500 | 500 |
| Squalane *Hydrophobic solvent* | | 5000 | 5000 | 5000 | 5000 | 5000 | 5000 | 5000 | 5000 | 5000 | 5000 | 5000 | 5000 |
| Polysorbate80 *High HLB emulsifier* | | 200 | 200 | 200 | 200 | 200 | 200 | 100 | 100 | 100 | 100 | 100 | 100 |
| | Sorbitan Monooleate *Low HLB emulsifier* | 500 | 600 | 700 | 800 | 900 | 1000 | 500 | 600 | 700 | 800 | 900 | 1000 |
| Stability | | Good | Good | Good | Good | Good | Good | Good | Good | Good | Good | Good | Good |

### EXAMPLE 8: Formulation Comprising Hydrophilic EGCG Compound in a Hydrophobic Squalane Solvent

About 4 grams of the hydrophilic EGCG compound was mixed with about 20 grams of propylene glycol (hydrophilic solvent). The mixture was heated to about 60°C until the hydrophilic EGCG compound was fully dissolved in the hydrophilic solvent to provide a EGCG solution (l'.e., hydrophilic phase) having a weight ratio of the hydrophilic EGCG compound to the hydrophilic solvent ofabout **1:5.**

To a stainless steel mortar and pestle, about 5 grams of squalane (or, alternatively, an equal amount of medicinal liquid paraffin) was added and heated to about 60° C. Then, about 2.7 grams of sorbitan monooleate surfactant having an HLB value of 4.3 (SPANTM 8O surfactant from Croda Inc.) was added and mixed well with the squalane.

About **500** mg of the EGCG solution (i.e., hydrophilic phase) and about 300 mg of polysorbate 80 surfactant having an HLB value of 15 (PEG-20 sorbitan monooleate, TWEEN^{®} 80 from Croda Inc.) were then added to the stainless steel mortar and pestle, and the mixture in the stainless steel mortar and pestle was mixed immediately. Then, the resulting mixture was allowed to cool down to room temperature under continued mixing to provide a formulation comprising about 1% by weight of the hydrophilic EGCG compound based on total weight of the formulation. After that, a portion of the formulation was transferred from the stainless steel mortar and pestle to a clear vial for a stability study. The formulation was a clear liquid having a light orange-beige color without opalescence or haze. The formulation showed an improved stability.

### EXAMPLE 9: Formulation Comprising Hydrophilic EGCG Compound in a Hydrophobic Squalane Solvent

About 1 gram of sunphenon EGCG was mixed with about 6 grams of propylene glycol (hydrophilic solvent). The mixture was heated to about 60°C until all solids were fully dissolved in the hydrophilic solvent to provide a EGCG solution (i.e. hydrophilic phase) having a weight ratio of the hydrophilic EGCG compound to the hydrophilic solvent of about **1:6.**

To this mixture, about 3 grams of TPGS were added. The resulting mixture was then mixed until it yielded a clear solution.

About 75 grams of squalane and about 15 grams of SPAN 80 were then combined in a main container and then mixed until they yielded a clear solution. The hydrophilic phase containing EGCG, propylene glycol, and TPGS was then added to the squalane-SPAN 80 mixture. The resulting mixture was then mixed until it yielded a clear solution. The formulation showed an improved stability. The ingredients may be mixed in the following percentages in the formulation: PROPYLENE GLYCOL **6.220,** SUNPHENON EGCG (TAIYO) **1.240,** TPGS -TOCOPHEROSAN (ISOLAN) **2.990,** SQUALANE **74.630,** and SPAN 80 (CRODA) **14.920.**

### EXAMPLE 10: Formulation Comprising Hydrophilic EGCG Compound in a Hydrophobic Drakeol 35 Solvent

About 1 gram of sunphenon EGCG was mixed with about 9 grams of propylene glycol (hydrophilic solvent). The mixture was heated to about 60°C until all solids were fully dissolved in the hydrophilic solvent to provide a EGCG solution (i.e. hydrophilic phase) having a weight ratio of the hydrophilic EGCG compound to the hydrophilic solvent of about 1:9.

To this mixture, about 4 grams of tocopherosan were added. The resulting mixture was then mixed until it yielded a clear solution.

About 110 grams of Drakeol 35 and about 21 grams of SPAN 80 were then combined in a main container and then mixed until they yielded a clear solution. The hydrophilic phase containing EGCG, propylene glycol, and tocopherosan was then added to the Drakeol 35-SPAN 80 mixture. The resulting mixture was then mixed until it yielded a clear solution. The formulation showed an improved stability.

The ingredients may be mixed in the following percentages in the formulation: PROPYLENE GLYCOL 6.220, SUNPHENON EGCG (TAIYO) 0.691, TOCOPHEROSAN (ISOCHEM) 2.990, DRAKEOL 35 (PENRECO), and SPAN 80 (CRODA) 14.920.

### EXAMPLE 11: Formulation Comprising Hydrophilic EGCG Compound in a Hydrophobic FINSOLV TN (c12-15 Alkyl Benzoate) Solvent

About 1 gram of sunphenon EGCG was mixed with about 9 grams of propylene glycol (hydrophilic solvent). The mixture was heated to about 60°C until all solids were fully dissolved in the hydrophilic solvent to provide a EGCG solution (i.e. hydrophilic phase) having a weight ratio of the hydrophilic EGCG compound to the hydrophilic solvent of about 1:9.

To this mixture, about 4 grams of tocopherosan were added. The resulting mixture was then mixed until it yielded a clear solution.

About 110 grams of FINSOLV TN and about 21 grams of SPAN 80 were then combined in a main container and then mixed until they yielded a clear solution. The hydrophilic phase containing EGCG, propylene glycol, and tocopherosan was then added to the FINSOLV TN-SPAN 80 mixture. The resulting mixture was then mixed until it yielded a clear solution. The formulation showed an improved stability.

The ingredients may be mixed in the following percentages in the formulation: PROPYLENE GLYCOL 6.220, SUNPHENON EGCG (TAIYO) 0.691, TOCOPHEROSAN (ISOCHEM) 2.990, FINSOLVTN (INNOSPEC) 75.179, and SPAN 80 (CRODA) 14.920.

### EXAMPLE 12: Formulation Comprising Hydrophilic EGCG Compound in a Hydrophobic Hemisqualane Solvent

About 1 gram of sunphenon EGCG was mixed with about 9 grams of propylene glycol (hydrophilic solvent). The mixture was heated to about 60°C until all solids were fully dissolved in the hydrophilic solvent to provide a EGCG solution (i.e. hydrophilic phase) having a weight ratio of the hydrophilic EGCG compound to the hydrophilic solvent of about 1:9.

To this mixture, about 4 grams of tocopherosan were added. The resulting mixture was then mixed until it yielded a clear solution.

About 110 grams of hemisqualane and about 21 grams of SPAN 80 were then combined in a main container and then mixed until they yielded a clear solution. The hydrophilic phase containing EGCG, propylene glycol, and tocopherosan was then added to the hemisqualane-SPAN 80 mixture. The resulting mixture was then mixed until it yielded a clear solution. The formulation showed an improved stability.

The formulation showed an improved stability. The ingredients may be mixed in the following percentages in the formulation: PROPYLENE GLYCOL 6.220, SUNPHENON EGCG (TAIYO) 0.691, TOCOPHEROSAN (ISOCHEM) 2.990, HEMISQUALANE 75.179, and SPAN 80 (CRODA) 14.920.

### EXAMPLE 13: Formulation Comprising Hydrophilic Centellin CG Compound in a Hydrophobic Squalane Solvent

About 1 gram of Centellin CG was mixed with about 19 grams of propylene glycol (hydrophilic solvent). The mixture was heated to about 60°C until all solids were fully dissolved in the hydrophilic solvent to provide a Centellin CG solution (i.e. hydrophilic phase) having a weight ratio of the hydrophilic Centellin CG compound to the hydrophilic solvent of about 1:19.

To this mixture, about 9 grams of tocopherosan were added. The resulting mixture was then mixed until it yielded a clear solution.

About 230 grams of squalane and about 45 grams of SPAN 80 were then combined in a main container and then mixed until they yielded a clear solution. The hydrophilic phase containing Centellin CG, propylene glycol, and tocopherosan was then added to the squalane-SPAN 80 mixture. The resulting mixture was then mixed until it yielded a clear solution. The formulation showed an improved stability. The ingredients may be mixed in the following percentages in the formulation: PROPYLENE GLYCOL 6.220, CENTELLIN CG (SABINSA) 0.330, TOCOPHEROSAN (ISOCHEM) 2.990, SQUALANE 75.410, and SPAN 80 (CRODA) 14.920.

### EXAMPLE 14: Formulation Comprising Hydrophilic Guarana Dry Extract Compound in a Hydrophobic Hemisqualane Solvent

About 1 gram of guarana dry extract was mixed with about 9 grams of propylene glycol (hydrophilic solvent). The mixture was heated to about 60°C until all solids were fully dissolved in the hydrophilic solvent to provide a Centellin CG solution (i.e. hydrophilic phase) having a weight ratio of the hydrophilic Centellin CG compound to the hydrophilic solvent of about 1:9.

To this mixture, about 4 grams of tocopherosan were added. The resulting mixture was then mixed until it yielded a clear solution.

About 110 grams of hemisqualane and about 22 grams of SPAN 80 were then combined in a main container and then mixed until they yielded a clear solution. The hydrophilic phase containing guarana dry extract, propylene glycol, and tocopherosan was then added to the hemisqualane-SPAN 80 mixture. The resulting mixture was then mixed until it yielded a clear solution. The formulation showed an improved stability.

The formulation showed an improved stability. The ingredients may be mixed in the following percentages in the formulation: PROPYLENE GLYCOL 6.220, GUARANA DRY EXTRACT (IDENA) .690, TOCOPHEROSAN (ISOCHEM) 2.940, HEMISQUALANE (NEOSSANCE) 75.150,and SPAN 80 (CRODA) 15.000.

Hydrophobic phase ingredients that did not form successful nanoemulsion formulations (generally began cloudy and separated into two phases, primarily within 24 hours) when substituted into the above examples include tocopheryl linoleate, olive oil, safflower oil, isopropyl palmitate, cetyl octanoate, octyl stearate, dimethicone, cycopentasiloxane, and ethylhexyl benzoate.

Hydrophobic phase ingredients that did form clear formulations were hydrocarbons (Squalene, Mineral Oil, c13-15 Alkane) and c12-15 Alkyl Benzoate. Hydrogenated polydecene, isododecane, isohexdecane, isoeicosane, and/or isoparaffins may substitute for squalane, mineral oil, c13-14 alkane, and/or c12-15 alkyl benzoate as hydrophobic phase ingredients in Examples 1-14 and yield mixtures with similarly improved stability.

Formulations include a hydrophilic phase, which includes a humectant

Certain features that are described in this specification in the context of separate embodiments also can be implemented in combination in a single embodiment. Conversely, various features that are described in the context of a single embodiment also can be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

## Claims

1. A formulation comprising: a continuous hydrophobic phase;
a hydrophilic phase dispersed throughout said continuous hydrophobic phase;
a stabilizing component comprising:
from 8% to 30% by weight D-a-tocopheryl polyethylene glycol 1000 succinate (TPGS) based upon total weight of the stabilizing component, and
from 70% to 92% by weight sorbitan monooleate having an HLB value of less than 10; and
a hydrophilic component comprising a compound that is normally insoluble in said continuous hydrophobic phase at room temperature absent an emulsifying agent,
wherein the hydrophilic component is substantially dissolved in the hydrophilic phase utilizing reverse micelles comprising the TPGS and the sorbitan monooleate, and
wherein the weight ratio of the stabilizing component to the hydrophilic component is at least 10:1 to 50:1.

2. The formulation of claim 1, wherein the hydrophilic component is present therein in an amount of at least 0.1% by weight based upon total weight of the formulation; or
wherein the stabilizing component is present in an amount of at least 15% by weight based upon total weight of the formulation; or
wherein the weight ratio of the continuous hydrophobic phase to the stabilizing component is no more than 5:1; or
wherein the TPGS is present in an amount of from 8% to 30% by weight based upon total weight of the stabilizing component, and the sorbitan monooleate has an HLB value of 3 to 8.

3. The formulation of claim 1,
wherein the hydrophilic component includes an ingredient selected from the group consisting of green tea extract, grape seed extract, fruit extract, centellin asiatica extract, guarana extract *(paullina cupana* seed extract) and polyphenol(s), or
wherein the hydrophilic component includes a compound selected from the group consisting of epigallocatechin gallate (EGCG), epigallocatechin (EGC), epicatechin (EC), epicatechin gallate (ECG), asiaticoside, madecassoside, asiatic acid, madecassic acid, caffeine, and catechin(s), or
wherein the hydrophilic component includes a compound selected from the group consisting of hydrolyzed pepper fruit extract *(Piper nigrum),* hydrolyzed soy protein, peppermint extract (*Mentha piperita*)*,* lotus (*Nelumbo nucifera*), coriander (*Coriandrum sativum*)*, Verbena officinalis* extract, and *Helianthus annuus* (sunflower) seed extract, or
wherein the hydrophilic component includes an ingredient selected from the group consisting of *Saccharomyces cerevisiae* extract, *Withania somnifera* root extract, butylene glycol and hydrolyzed *Coriandrum sativum* fruit extract and *Citrus aurantium dulcis* (orange) fruit extract, *Helianthus annuus* (sunflower) seed extract, *Taraxacum of ficinale* (dandelion) extract, hydrolyzed *Viola tricolor* extract, *Pyrus malus* (apple) fruit extract, hydrolyzed *Celosia cristata* extract and hydrolyzed *Prune la vulgaris* extract, hydrolyzed *Citrus aurantium dulcis* fruit extract, hydrolyzed *Candida saitoana* extract, *Lindera strychnifolia* root extract, *Triticum vulgare* (wheat germ) extract, *Secale cereale* (rye) seed extract, hydrolyzed sesame extract, *Tropaeol um majus* flower extract, *Spiraea ulmaria* extract, hydrolyzed sweet almond protein, *Salix Alba* (willow) leaf extract, *Mentha piperita* (peppermint) extract, *Triticum vulgare* (wheat) germ extract, butylene glycol and *Butyrospermum parlzii* (shea butter) seedcake extract, hydrolyzed lupine protein octenylsuccinate, hydrolyzed *Cucurbita pepo* (pumpkin) seedcake, hydrolyzed *Opuntia ficus indica* flower extract, *Medicago sativa* (alfalfa) seed extract and hydrolyzed lupine protein, *Lentinus edodes* extract, hydrolyzed *Ceratonia siliqua* seed extract, *Gossypium hirsutum* (cotton) extract, *Helianthus annuus* (sunflower) seed extract, *Jasminum officinale* (jasmine) flower extract, hydrolyzed *Manihot esculenta* tuber extract, butylene glycol and *Iris florentina* root extract, hydrolyzed wheat protein, hydrolyzed *Myrtus communis* leaf extract, butylene glycol and *Boerhavia diffusa* root extract, *Cynara scolymus* (artichoke) leaf extract, yeast extract, hydrolyzed rice protein, *Nasturtium officinale* extract, *Avena sativa* (oat) kernel extract, *Tropaeolum majus* flower/leaf/stem extract, *Lens esculenta* (lentil) seed extract (including oligosaccharides thereof), *Cyperus esculentus* tuber extract, *Prunus amygdalus dulcis* (sweet almond) seed extract, hydrolyzed soy fiber, *Pichia anomala* extract, butylene glycol and *Nelumbo nucifera* leaf extract, yeast extract, butylene glycol and *Artemisia abrotanum* extract, hydrolyzed soy flour, *Castanea sativa* (chestnut) seed extract, propylene glycol and *Citrus aurantium amara* (bitter orange) flower extract, hydrolyzed pepper fruit extract, hydrolyzed soy protein, butylene glycol and *Spiraea ulmaria* extract, hydrolyzed linseed extract, butylene glycol and *Peumus boldus* leaf extract, red algae (*Kappaphycus alvarezii*) galactans, *Pichia anomala* (including biopeptides thereof) extract, sulfated galactans of *Hypnea musciformis, Glycine soja* hydrolyzed soy protein, *Oryza sativa* extract, *Cyperus esculentus* tuber extract, Piper nigrum hydrolyzed pepper fruit extract, *Verbena officinalis* extract, butylene glycol and *Peumus boldus* leaf extract, butylene glycol and *Peumus boldus* leaf extract, seed extract, hydrolyzed *Cicer* seed extract, *Triticum Vulgare* (wheat) germ extract, hydrolyzed pea, butylene glycol and *Silybum Marianum* extract, hydrolyzed lupine protein, *Kappaphycus alvarezii* (red algae) extract, *Triticum vulgare* (wheat) protein, yeast extract, *Cichorium intybus* (chicory) root extract, *Prunus persica* (peach) leaf extract, *Verbena officinalis* extract, *Medicago sativa* (alfalfa) extract, *Paeonia albiflora* root extract, *Palmaria palmata* extract, hydrolyzed millet, butylene glycol and triethanolamine and *Punica granatum* extract, glycerin and *Prunus amygdalus dulcis* (sweet almond) extract, hydrolyzed sweet almond seedcake, butylene glycol and *Fumaria officinalis* extract, butylene glycol and *Camellia sinensis* leaf extract, and soytrimonium chloride, or
wherein the hydrophilic component includes a compound selected from the group consisting of a peptide, an amino acid, a polynucleotide, and a hydrophilic drug.

4. The formulation of claim 1, wherein the hydrophilic phase includes at least one solvent selected from the group consisting of water, ethanol, dimethyl sulfoxide, propylene glycol, and polyethylene glycol, or
wherein the continuous hydrophobic phase comprises a solvent selected from the group consisting of mineral oil, vegetable oil, paraffin, polysiloxane, cyclopentasiloxane, dimethiconol, squalane, C12-15 alkyl benzoate (such as Finsolv TN), C13-15 alkane (such as hemisqualane) and mixtures thereof.

5. The formulation of claim 1, wherein the hydrophilic component comprises a green tea extract, centella asiatica extract, and/ or guarana extract in an amount of at least 0.1% by weight based upon total weight of the formulation; and
the stabilizing component is present in an amount of at least 15% by weight based upon total weight of the formulation, the stabilizing component consisting of from 8% to 30% by weight TPGS based upon total weight of the stabilizing component and from 70% to 92% by weight sorbitan monooleate having an HLB value of 3 to 8 based upon total weight of the stabilizing component,
wherein a weight ratio of the stabilizing component to the green tea extract is at least 10:1.

6. A method of preparing the formulation of claim 1, the method comprising: dissolving the hydrophilic component in the hydrophilic phase to provide a solution of the hydrophilic component;
adding TPGS emulsifier to the solution of hydrophilic component; and then mixing with a mixture comprising the continuous hydrophobic phase and the emulsifier(s) having an HLB value of less than 10 so as to provide the formulation.

7. Formulation as defined in claim 1 for use as a medicament.

8. The formulation of claim 1, wherein, at room temperature, the formulation exists as a nanoemulsion with the hydrophilic component dispersed throughout the formulation, but, upon application to a subject's skin, with the concomitant temperature change caused thereby, the nanoemulsion disrupts and the formulation directly deposits the hydrophilic component onto the subject's skin, or
wherein the hydrophilic component includes a collagen suppressing compound, in particular wherein the collagen suppressing compound is a bicyclooctanone.

9. Formulation of claim 1 for use in a method of suppressing keloid formation in a subject undergoing surgery , in particular wherein the formulation is applied to an incision site for a finite number of days after surgery.

## Patentansprüche

1. Formulierung, umfassend: eine kontinuierliche hydrophobe Phase;
eine hydrophile Phase, die in der gesamten kontinuierlichen hydrophoben Phase aufgelöst ist;
eine Stabilisierungskomponente, umfassend:
8 Gew.-% bis 30 Gew.-% D-a-Tocopherylpolyethylenglycol-1000-succinat (TPGS) basierend auf dem Gesamtgewicht der Stabilisierungskomponente und
70 Gew.-% bis 92 Gew.-% Sorbitanmonooleat mit einem HLB-Wert von weniger als 10; und
eine hydrophile Komponente, die eine Verbindung umfasst, die in der kontinuierlichen hydrophoben Phase bei Raumtemperatur ohne Emulgierungsmittel normalerweise unlöslich ist,
wobei die hydrophile Komponente im Wesentlichen unter Verwendung von Umkehrmizellen, die das TPGS und das Sorbitanmonooleat umfassen, in der hydrophilen Phase aufgelöst wird, und
wobei das Gewichtsverhältnis der Stabilisierungskomponente zu der hydrophilen Komponente mindestens 10:1 bis 50:1 beträgt.

2. Formulierung nach Anspruch 1, wobei die hydrophile Komponente darin in einer Menge von mindestens 0,1 Gew.-% basierend auf dem Gesamtgewicht der Formulierung vorhanden ist; oder
wobei die Stabilisierungskomponente in einer Menge von mindestens 15 Gew.-% basierend auf dem Gesamtgewicht der Formulierung vorhanden ist; oder
wobei das Gewichtsverhältnis der kontinuierlichen hydrophoben Phase zu der Stabilisierungskomponente nicht mehr als 5:1 beträgt; oder
wobei das TPGS in einer Menge von 8 Gew.-% bis 30 Gew.-% basierend auf dem Gesamtgewicht der Stabilisierungskomponente vorhanden ist und das Sorbitanmonooleat einen HLB-Wert von 3 bis 8 aufweist.

3. Formulierung nach Anspruch 1,
wobei die hydrophile Komponente einen Inhaltsstoff beinhaltet, der ausgewählt ist aus der Gruppe bestehend aus Grünteeextrakt, Traubenkernextrakt, Fruchtextrakt, *Centella-asiatica*-Extrakt, Guarana-Extrakt (*Paullinia-cupana-*Kernextrakt) und (einem) Polyphenol(en), oder
wobei die hydrophile Komponente eine Verbindung beinhaltet, die ausgewählt ist aus der Gruppe bestehend aus Epigallocatechingallat (EGCG), Epigallocatechin (EGG), Epicatechin (EC), Epicatechingallat (ECG), Asiaticosiden, Madecassosiden, Asiaticasäure, Madecassinsäure, Koffein und Catechin(en), oder
wobei die hydrophile Komponente eine Verbindung beinhaltet, die ausgewählt ist aus der Gruppe bestehend aus hydrolysiertem Pfefferfruchtextrakt (*Piper nigrum*), hydrolysiertem Sojaprotein, Pfefferminzextrakt (*Mentha piperita*), Lotus (*Nelumbo nucifera*), Koriander (*Coriandrum sativum*), *Verbena-officinalis-Extrakt* und *Helianthus-annuus*-(Sonnenblumen-)Kernextrakt, oder
wobei die hydrophile Komponente einen Inhaltsstoff beinhaltet, der ausgewählt ist aus der Gruppe bestehend aus *Saccharomyces-cerevisiae-Extract, Withania-somnifera-*Wurzelextrakt, Butylenglycol und hydrolysiertem *Coriandrum*sativum-Fruchtextrakt und *Citrus-aurantium-dulcis*-(Orangen-)Fruchtextrakt, *Helianthus-annuus*-(Sonnenblumen-)Kernextrakt, *Taraxacum-officinale-*(Löwenzahn-)Extrakt, hydrolysiertem *Violatricolor*-Extrakt, *Pyrus*-malus-(Apfel-)Fruchtextrakt, hydrolysiertem *Celosia-cristata*-Extrakt und hydrolysiertem *Prunella-vulgaris-*Extrakt, hydrolysiertem *Citrus-aurantiumdulcis*-Fruchtextrakt, hydrolysiertem *Candida-saitoana-*Extrakt, *Lindera-strychnifolia-Wurzelextrakt, Triticum-vulgare-*(Weizenkeim-)Extrakt, *Secale-cereale*-(Roggen-)Kernextrakt, hydrolysiertem Sesamextrakt, *Tropaeolum-majus*-Blumenextrakt, *Spiraea-ulmaria-*Extrakt, hydrolysiertem Süßmandelprotein, *Salix-alba*-(Weiden-)Blattextrakt, *Mentha-piperita-*(Pfefferminz-)Extrakt, *Triticum-vulgare*-(Weizenkeim-)Extrakt, Butylenglycol und *Butyrospermum-parkii*-(Sheabutter-)Kernextrakt, hydrolysiertem Lupinenprotein-Octenylsuccinat, hydrolysiertem *Cucurbita-pepo*-(Kürbis-)Kern, hydrolysiertem *Opuntia-flcus-indica*-Blumenextrakt, *Medicago-sativa*-(Alfalfa-)Kernextrakt und hydrolysiertem Lupinenprotein, *Lentinus*edodes-Extrakt, hydrolysiertem *Ceratonia-siliqua*-Kernextrakt, *Gossypium-hirsutum-(Baumwolle-)Extrakt, Helianthus-annuus-*(Sonnenblumen-)Kernextrakt, *Jasminum-officinale*-(Jasmin-)Blumenextrakt, hydrolysiertem *Manihot-esculenta-*Knollenextrakt, Butylenglycol und *Iris-florentina*-Wurzelextrakt, hydrolysiertem Weizenprotein, hydrolysiertem *Myrtus-communis-*Blattextrakt, Butylenglycol und *Boerhavia-diffusa-*Wurzelextrakt, *Cynara*-scolymus-(Artischocken-)Blattextrakt, Hefeextrakt, hydrolysiertem Reisprotein, *Nasturtium-officinale-*Extrakt, Avena-sativa-(Hafer-)Kornextrakt, *Tropaeolum-majus-*Blumen-/Blatt-/Stielextrakt, *Lens-esculenta*-(Linsen-)Kernextrakt (einschließlich Oligosaccharide davon), *Cyperus-*esculentus-Knollenextrakt, *Prunus-amygdalus-dulcis*-(Süßmandel-)Kernextrakt, hydrolysierter Sojafaser, *Pichia-anomala*-Extrakt, Butylenglycol und *Nelumbo-nucifera*-Blattextrakt, Hefeextrakt, Butylenglycol und *Artemisia-abrotanum*-Extrakt, hydrolysiertem Sojamehl, *Castanea-sativa*-(Kastanien-)Kernextrakt, Propylenglycol und *Citrus-aurantium-amara*-(Bitterorangen-)Blumenextrakt, hydrolysiertem Pfefferfruchtextrakt, hydrolysiertem Sojaprotein, Butylenglycol und *Spiraea-ulmaria-*Extrakt, hydrolysiertem Leinkernextrakt, Butylenglycol und *Peumus-boldus*-Blattextrakt, Rotalgen-(*Kappaphycus-alvarezii-*)Galactane, *Pichia-anomala*-Extrakt (einschließlich Biopeptide davon), sulfatierter Galactane von *Hypnea musciformis,* hydrolysiertem Sojaprotein von *Glycine soja, Oryza-sativa-*Extrakt, *Cyperus*-esculentus-Knollenextrakt, hydrolysiertem Pfefferfruchtextrakt von *Piper nigrum, Verbena-officinalis-*Extrakt, Butylenglycol and *Peumus-boldus*-Blattextrakt, Butylenglycol und *Peumus-boldus*-Blattextrakt, Kernextrakt, hydrolysiertem *Cicer*-Kernextrakt, *Triticum-vulgare*-(Weizen-)Keimextrakt, hydrolysierter Erbse, Butylenglycol und *Silybummarianum-*Extrakt, hydrolysiertem Lupinenprotein, *Kappaphycus-alvarezii*-(Rotalgen-)Extrakt, *Triticum-vulgare*-(Weizen-)Protein, Hefeextrakt, *Cichorium-intybus*-(Chicorée-)Wurzelextrakt, *Prunus-persica*-(Pfirsich-)Blattextrakt, *Verbena-officinalis-Extrakt, Medicago-sativa*-(Alfalfa-)Extrakt, *Paeonia-albiblora*-Wurzelextrakt, *Palmaria-palmata-*Extrakt, hydrolysierter Hirse, Butylenglycol und Triethanolamin und *Punica-granatum*-Extrakt, Glycerin und *Prunus-amygdalus-dulcis*-(Süßmandel-)Extrakt, hydrolysiertem Süßmandelkern, Butylenglycol und *Fumaria-officinalis*-Extrakt, Butylenglycol und *Camellia-sinensis*-Blattextrakt und Sojatrimoniumchlorid, oder
wobei die hydrophile Komponente eine Verbindung beinhaltet, die ausgewählt ist aus der Gruppe bestehend aus einem Peptid, einer Aminosäure, einem Polynukleotid und einem hydrophilen Arzneimittel.

4. Formulierung nach Anspruch 1, wobei die hydrophile Phase mindestens ein Lösungsmittel beinhaltet, das ausgewählt ist aus der Gruppe bestehend aus Wasser, Ethanol, Dimethylsulfoxid, Propylenglycol und Polyethylenglycol, oder
wobei die kontinuierliche hydrophobe Phase ein Lösungsmittel umfasst, das ausgewählt ist aus der Gruppe bestehend aus Mineralöl, Pflanzenöl, Paraffin, Polysiloxan, Cyclopentasiloxan, Dimethiconol, Squalan, C12-15-Alkylbenzoat (wie etwa Finsolv TN), C13-15-Alkan (wie etwa Hemisqualan) und Gemischen davon.

5. Formulierung nach Anspruch 1, wobei die hydrophile Komponente einen Grünteeextrakt, Centella-asiatica-Extrakt und/oder Guaranaextrakt in einer Menge von mindestens 0,1 Gew.-% basierend auf dem Gesamtgewicht der Formulierung umfasst; und
die Stabilisierungskomponente in einer Menge von mindestens 15 Gew.-% basierend auf dem Gesamtgewicht der Formulierung vorhanden ist, wobei die Stabilisierungskomponente aus 8 Gew.-% bis 30 Gew.-% TPGS basierend auf dem Gesamtgewicht der Stabilisierungskomponente und 70 Gew.-% bis 92 Gew.-% Sorbitanmonooleat mit einem HLB-Wert von 3 bis 8 basierend auf dem Gesamtgewicht der Stabilisierungskomponente besteht,
wobei ein Gewichtsverhältnis der Stabilisierungskomponente zu dem Grünteeextrakt mindestens 10:1 beträgt.

6. Verfahren zum Herstellen der Formulierung nach Anspruch 1, wobei das Verfahren Folgendes umfasst: Auflösen der hydrophilen Komponente in der hydrophilen Phase, um eine Lösung der hydrophilen Komponente bereitzustellen;
Hinzufügen eines TPGS-Emulgators zu der Lösung der hydrophilen Komponente; und dann Mischen mit einem Gemisch, das die kontinuierliche hydrophobe Phase und den/die Emulgator(en) mit einem HLB-Wert von weniger als 10 umfasst, um die Formulierung bereitzustellen.

7. Formulierung nach Anspruch 1 zur Verwendung als Medikament.

8. Formulierung nach Anspruch 1, wobei die Formulierung bei Raumtemperatur als Nanoemulsion vorhanden ist, wobei die hydrophile Komponente in der gesamten Formulierung aufgelöst ist, jedoch bei Auftragen auf die Haut eines Patienten mit dadurch verursachter gleichzeitiger Temperaturänderung, oder
wobei die hydrophile Komponente eine Kollagen unterdrückende Verbindung beinhaltet, wobei insbesondere die Kollagen unterdrückende Verbindung ein Bicyclooctanon ist.

9. Formulierung nach Anspruch 1 zur Verwendung in einem Verfahren zum Unterdrücken einer Keloidbildung bei einem Patienten bei einer Operation, wobei insbesondere die Formulierung auf eine Inzisionsstelle für eine endliche Anzahl von Tagen nach der Operation aufgetragen wird.

## Revendications

1. Formulation comprenant : une phase continue hydrophobe ;
une phase hydrophile dispersée dans toute ladite phase hydrophobe continue ;
un composant stabilisant comprenant :
de 8 % à 30 % en poids de succinate acide de d-alpha-tocophéryle (TPGS) 1000 par rapport au poids total du composant stabilisant, et
de 70 % à 92 % en poids de monooléate de sorbitan ayant une valeur HLB inférieure à 10 ; et
un composant hydrophile comprenant un composé qui est normalement insoluble dans ladite phase hydrophobe continue à température ambiante dépourvue d'agent émulsifiant,
dans laquelle le composant hydrophile est sensiblement dissous dans la phase hydrophile en utilisant des micelles inverses comprenant le TPGS et le monooléate de sorbitan, et
dans laquelle le rapport pondéral du composant stabilisant au composant hydrophile est d'au moins 10:1 à 50:1.

2. Formulation selon la revendication 1, dans laquelle le composant hydrophile est présent à l'intérieur en une quantité d'au moins 0,1 % en poids sur la base du poids total de la formulation ; ou
dans laquelle le composant stabilisant est présent en une quantité d'au moins 15 % en poids sur la base du poids total de la formulation ; ou
dans laquelle le rapport pondéral de la phase hydrophobe continue au composant stabilisant n'est pas supérieur à 5:1 ; ou
dans laquelle le TPGS est présent en une quantité de 8 à 30 % en poids sur la base du poids total du composant stabilisant, et le monooléate de sorbitan a une valeur HLB de 3 à 8.

3. Formulation selon la revendication 1,
dans laquelle le composant hydrophile comporte un ingrédient choisi dans le groupe constitué d'extrait de thé vert, d'extrait de pépins de raisin, d'extrait de fruit, d'extrait de Centella Asiatica, d'extrait de guarana (extrait de graine de *paullinia cupana*) et de polyphénol(s), ou
dans laquelle le composant hydrophile comporte un composé choisi dans le groupe constitué du gallate d'épigallocatéchine (EGCG), de l'épigallocatéchine (EGC), de l'épicatéchine (EC), le gallate d'épicatéchine (ECG), de l'asiaticoside, du madécassoside, de l'acide asiatique, de l'acide madécassique, de la caféine et de catéchine(s), ou
dans laquelle le composant hydrophile comporte un composé choisi dans le groupe constitué d'extrait de fruit de poivre hydrolysé (*Piper nigrum*), de protéine de soja hydrolysée, d'extrait de menthe poivrée (*Mentha piperita*), de lotus (*Nelumbo nucifera*), de coriandre (*Coriandrum sativum*), d'extrait de *Verbena officinalis,* et d'extrait de graine d'*Helianthus annuus* (tournesol), ou
dans laquelle le composant hydrophile comporte un ingrédient choisi dans le groupe constitué d'extrait de *Saccharomyces cerevisiae*, d'extrait de racine de *Withania somnifera,* d'extrait de butylène glycol et de fruit de *Coriandrum sativum* hydrolysé et d'extrait de fruit de *Citrus aurantium dulcis* (orange), d'extrait de graine *d'Helianthus annuus* (tournesol), d'extrait de *Taraxacum officinale* (pissenlit), d'extrait de *Viola tricolor* hydrolysé, d'extrait de fruit de *Pyrus malus* (pomme), d'extrait de *Celosia cristata* hydrolysé et d'extrait de *Prune la vulgaris* hydrolysé, d'extrait de fruit de *Citrus aurantium dulcis* hydrolysé, d'extrait de *Candida saitoana* hydrolysé, d'extrait de racine de *Lindera strychnifolia,* d'extrait de *Triticum vulgare* (germe de blé), d'extrait de graine de *Secale cereale* (seigle), d'extrait de sésame hydrolysé, d'extrait de fleur de *Tropaeol um majus,* d'extrait de *Spiraea ulmaria,* de protéine d'amande douce hydrolysée, d'extrait de feuille de *Salix Alba* (saule), d'extrait de *Mentha piperita* (menthe poivrée), d'extrait de germe de *Triticum vulgare* (blé), d'extrait de tourteau de butylène glycol et de *Butyrospermum parlzii* (beurre de karité), d'octénylsuccinate de protéine de lupin hydrolysée, de tourteau de graine de *Cucurbita pepo* (citrouille) hydrolysé, d'extrait de fleur *d'Opuntia ficus indica* hydrolysé, d'extrait de graine de *Medicago sativa* (alfalfa) et de protéine de lupin hydrolysée, d'extrait de *Lentinus edodes,* d'extrait de graine de *Ceratonia siliqua* hydrolysé, d'extrait de *Gossypium hirsutum* (coton), d'extrait de graine d' *Helianthus annuus* (tournesol), d'extrait de fleur de *Jasminum officinale* (jasmin), d'extrait de tubercule de *Manchot esculenta* hydrolysé, d'extrait de racine de butylène glycol et d'*Iris florentina,* de protéine de blé hydrolysée, d'extrait de feuille de *Myrtus communis* hydrolysée, d'extrait butylène glycol et de racine de *Boerhavia diffusa,* d'extrait de feuille de *Cynara scolymus* (artichaut), d'extrait de levure, de protéine de riz hydrolysée, d'extrait de *Nasturtium officinale,* d'extrait de noyau d'*Avena sativa* (avoine), d'extrait de fleur/feuille/tige de *Tropaeolum majus,* d'extrait de graine de *Lens esculenta* (lentille) (y compris ses oligosaccharides), d'extrait de tubercule de *Cyperus esculentus,* d'extrait de graine de *Prunus amygdalus dulcis* (amande douce), de fibre de soja hydrolysée, d'extrait de *Pichia anomala,* d'extrait de butylène glycol et de feuille de *Nelumbo nucifera,* d'extrait de levure, d'extrait de butylène glycol et d'*Artemisia abrotanum,* de farine de soja hydrolysée, d'extrait de graine de *Castanea sativa* (châtaigne), d'extrait de propylène glycol et de fleur de *Citrus aurantium amara* (orange amère), d'extrait de fruit de poivre hydrolysé, de protéine de soja hydrolysée, d'extrait de butylène glycol et de *Spiraea ulmaria,* d'extrait de graine de lin hydrolysées, d'extrait de butylène glycol et de feuille de *Peumus boldus,* de galactanes d'algue rouge (*Kappaphycus alvarezii*)*,* d'extrait de *Pichia anomala* (y compris ses biopeptides), de galactanes sulfatés *d'Hypnea musciformis,* protéine de soja hydrolysée *Glycine soja,* d'extrait *d'Oryza sativa,* d'extrait de tubercule de *Cyperus esculentus,* d'extrait de fruit de poivre hydrolysé de Piper nigrum, d'extrait de *Verbena officinalis,* d'extrait de butylène glycol et de feuille de *Peumus boldus,* d'extrait de butylène glycol et de feuille de *Peumus boldus,* d'extrait de graine, d'extrait de graine de *Cicer* hydrolysé, d'extrait de germe de *Triticum Vulgare* (blé), de pois hydrolysé, d'extrait de butylène glycol et de *Silybum Marianum,* de protéine de lupin hydrolysée, d'extrait de *Kappaphycus alvarezii* (algue rouge), de protéine de *Triticum vulgare* (blé), d'extrait de levure, d'extrait de racine de *Cichorium intybus* (chicorée), d'extrait de feuille de *Prunus persica* (pêche), d'extrait de *Verbena officinalis,* d'extrait de *Medicago sativa* (luzerne), d'extrait de racine de *Paeonia albiflora,* d'extrait de *Palmaria palmata,* de millet hydrolysé, d'extrait butylène glycol et de triéthanolamine et de *Punica granatum,* d'extrait de glycérine et de *Prunus amygdalus dulcis* (amande douce), tourteau d'amande douce hydrolysé, d'extrait de butylène glycol et de *Fumaria officinalis,* d'extrait de butylène glycol et de feuille de *Camellia sinensis,* et de chlorure de soytrimonium, ou
dans laquelle le composant hydrophile comporte un composé choisi dans le groupe constitué d'un peptide, d'un acide aminé, d'un polynucléotide et d'un médicament hydrophile.

4. Formulation selon la revendication 1, dans laquelle la phase hydrophile comporte au moins un solvant choisi dans le groupe constitué par l'eau, l'éthanol, du diméthylsulfoxyde, du propylène glycol et du polyéthylène glycol, ou
dans laquelle la phase hydrophobe continue comprend un solvant choisi dans le groupe constitué de l'huile minérale, de l'huile végétale, de la paraffine, du polysiloxane, du cyclopentasiloxane, du diméthiconol, du squalane, du benzoate d'alkyle en C12-15 (tel que Finsolv TN), de l'alcane en C13-15 (tel que l'hémisqualane) et leurs mélanges.

5. Formulation selon la revendication 1, dans laquelle le composant hydrophile comprend un extrait de thé vert, un extrait de centella asiatica et/ou un extrait de guarana en une quantité d'au moins 0,1 % en poids sur la base du poids total de la formulation ; et
le composant stabilisant est présent en une quantité d'au moins 15 % en poids sur la base du poids total de la formulation, le composant stabilisant étant constitué de 8 % à 30 % en poids de TPGS sur la base du poids total du composant stabilisant et de 70 % à 92 % en poids de monooléate de sorbitan ayant une valeur HLB de 3 à 8 sur la base du poids total du composant stabilisant,
dans laquelle un rapport pondéral du composant stabilisant à l'extrait de thé vert est d'au moins 10:1.

6. Procédé de préparation de la formulation selon la revendication 1, le procédé comprenant : la dissolution du composant hydrophile dans la phase hydrophile pour fournir une solution du composant hydrophile ;
l'ajout de l'émulsifiant TPGS à la solution du composant hydrophile ; puis le mélange avec un mélange comprenant la phase continue hydrophobe et le ou les émulsifiant(s) ayant une valeur HLB inférieure à 10 pour fournir la formulation.

7. Formulation telle que définie dans la revendication 1 pour une utilisation en tant que médicament.

8. Formulation selon la revendication 1, dans laquelle, à température ambiante, la formulation existe sous la forme d'une nanoémulsion avec le composant hydrophile dispersé dans toute la formulation, mais, lors de l'application sur la peau d'un sujet, avec le changement de température concomitant provoqué par celle-ci, la nanoémulsion perturbe et la formulation dépose directement le composant hydrophile sur la peau du sujet, ou
dans laquelle le composant hydrophile comporte un composé supprimant le collagène, en particulier dans laquelle le composé supprimant le collagène est une bicyclooctanone.

9. Formulation selon la revendication 1 pour une utilisation dans un procédé de suppression de la formation de chéloïdes chez un sujet subissant une intervention chirurgicale, en particulier dans laquelle la formulation est appliquée sur un site d'incision pendant un nombre fini de jours après l'intervention chirurgicale.
